# EUROPEAN PATENT SPECIFICATION

(11) **EP 1 761 647 B1**
(45) Date of publication and mention of the grant of the patent: **02.12.2009**
(21) Application number: 05762191.4
(22) Date of filing: 21.06.2005
(51) Int. Cl.: C12Q 1/68

(54) **TWO-HYBRID SYSTEM**
ZWEIFACHES HYBRIDSYSTEM
SYSTEME A DEUX HYBRIDES

(30) Priority: 30.06.2004 US 882996
(43) Date of publication of application: 14.03.2007
(73) Proprietor: Applied Biosystems, LLC, Carlsbad, CA 92008 (US)
(72) Inventor: FUERNKRANZ, Hans, A., Saratoga, CA 95070 (US); TAN, Roy, H., Union City, CA 94587 (US)
(74) Representative: Grund, Martin
(86) International application number: PCT/US2005/021856
(87) International publication number: WO 2006/012105

(56) References cited:
- WO-A-03/031591
- US-A- 5 726 024
- ZHU HENG ET AL: "Proteomics." ANNUAL REVIEW OF BIOCHEMISTRY. 2003, vol. 72, 2003, pages 783-812, XP009053823 ISSN: 0066-4154
- SCHWEITZER BARRY ET AL: "Microarrays to characterize protein interactions on a whole-proteome scale." PROTEOMICS. NOV 2003, vol. 3, no. 11, November 2003 (2003-11), pages 2190-2199, XP009053822 ISSN: 1615-9853
- HO Y ET AL: "Systematic identification of protein complexes in Saccharomyces cerevisiae by mass spectrometry" NATURE, MACMILLAN JOURNALS LTD. LONDON, GB, vol. 415, 10 January 2002 (2002-01-10), pages 180-183, XP002250082 ISSN: 0028-0836
- GAVIN A-C ET AL: "Functional organization of the yeast proteome by systematic analysis of protein complexes" NATURE, MACMILLAN JOURNALS LTD. LONDON, GB, vol. 415, January 2002 (2002-01), pages 141-147, XP002958851 ISSN: 0028-0836
- NUWAYSIR EMILE F ET AL: "Gene expression analysis using oligonucleotide arrays produced by maskless photolithography." GENOME RESEARCH. NOV 2002, vol. 12, no. 11, November 2002 (2002-11), pages 1749-1755, XP009053945 ISSN: 1088-9051

## Description

### Field

Methods of detecting targets in a sample are provided. Methods of quantitating targets in a sample are provided. Methods of determining the specificity of a first molecule for a second molecule are provided. Methods of detecting inhibitors or enhancers of an interaction between a first molecule and a second molecule are provided. Methods of measuring the affinity of two different molecules for a different third molecule are provided.

### Background

A two-hybrid system is useful for detecting and/or quantitating probe-target interactions. In certain instances, a two-hybrid system is used to detect interactions between a known probe and an unknown target. In certain instances, a two-hybrid system is used to quantitate the strength of interaction between a probe and a target. The interactions may be detected, for example, by linking a detecting bead to the target and a separating bead to the probe. In certain instances, where individual interactions can be counted, a two-hybrid system can quantitate the strength of interactions that occur between a probe and a target. Furthermore, in certain instances, a two-hybrid system may be used to simultaneously detect multiple different interactions between probes and targets in the same assay.

### SUMMARY

The invention relates to methods as defined in the appended claims. In certain embodiments, a method for detecting at least one target is provided. In certain embodiments, a reaction composition is formed comprising a target group and a probe set, wherein the target group comprises a plurality of target sets. In certain embodiments, each target set of the target group comprises: (a) a polypeptide comprising a target set nucleic acid binding domain and a target, and (b) a target set nucleic acid comprising a target set polypeptide binding sequence. In certain embodiments, the target set polypeptide binding sequence is capable of binding to the target set nucleic acid binding domain. In certain embodiments, the target group comprises a plurality of target sets that comprise the same target set nucleic acid binding domain and the same target set nucleic acid and comprise different targets. In certain embodiments, the probe set comprises: (a) a polypeptide comprising a probe set nucleic acid binding domain and a probe, and (b) a probe set nucleic acid comprising a probe set polypeptide binding sequence. In certain embodiments, the probe set polypeptide binding sequence is capable of binding to the probe set nucleic acid binding domain. In certain embodiments, at least one of the target set nucleic acid and the probe set nucleic acid comprises at least one addressable portion. In certain embodiments, the reaction composition is incubated under reaction conditions such that if the probe interacts with a target of a target set, a binding complex is produced, wherein the binding complex comprises the probe set and the target set. In certain embodiments, a label associated with the binding complex is detected using at least one of the at least one addressable portions. In certain embodiments, the at least one target is detected by detecting the label.

In certain embodiments, a method for detecting at least one interaction between a probe and a target is provided. In certain embodiments, a reaction composition is formed comprising a target set and a probe set. In certain embodiments, the target set comprises: (a) a polypeptide comprising a target set nucleic acid binding domain and the target, and (b) a target set nucleic acid comprising a target set polypeptide binding sequence, wherein the target set polypeptide binding sequence is capable of binding to the target set nucleic acid binding domain. In certain embodiments, the probe set comprises: (a) a polypeptide comprising a probe set nucleic acid binding domain and the probe, and (b) a probe set nucleic acid comprising a probe set polypeptide binding sequence, wherein the probe set polypeptide binding sequence is capable of binding to the probe set nucleic acid binding domain. In certain embodiments, at least one of the target set nucleic acid and the probe set nucleic acid comprises at least one addressable portion. In certain embodiments, the reaction composition is incubated under reaction conditions such that if the probe interacts with the target, a binding complex is produced, wherein the binding complex comprises the probe set and the target set. In certain embodiments, a label associated with the binding complex is detected using at least one of the at least one addressable portions. In certain embodiments, the at least one interaction between the probe and the target is detected by detecting the label.

In certain embodiments, a method for detecting at least one target is provided. In certain embodiments, a reaction composition is formed comprising one or more target groups and one or more probe sets, wherein each target group comprises one or more target sets. In certain embodiments, each target set of each target group comprises: (a) a polypeptide comprising a target set nucleic acid binding domain and a target, and (b) a target set nucleic acid comprising a target set polypeptide binding sequence, wherein the target set polypeptide binding sequence is capable of binding to the target set nucleic acid binding domain. In certain embodiments, if a target group comprises multiple different target sets, the target group comprises a plurality of target sets that comprise the same target set nucleic acid binding domain and the same target set nucleic acid and comprise different targets. In certain embodiments, each probe set comprises: (a) a polypeptide comprising a probe set nucleic acid binding domain and a probe, and (b) a probe set nucleic acid comprising a probe set polypeptide binding sequence, wherein the probe set polypeptide binding sequence is capable of binding to the probe set nucleic acid binding domain. In certain embodiments, at least one of the one or more target set nucleic acids and the one or more probe set nucleic acids comprises at least one addressable portion. In certain embodiments, the reaction composition is incubated under reaction conditions such that if a probe of a probe set interacts with a target of a target set, a binding complex is produced, wherein the binding complex produced comprises the probe set and the target set. In certain embodiments, a label associated with the binding complex is detected using at least one of the at least one addressable portions. In certain embodiments, the at least one target is detected by detecting the label.

In certain embodiments, a method for comparing the binding affinities of two different targets with a probe is provided. In certain embodiments, a reaction composition is formed comprising two different target sets and a probe set. In certain embodiments, the first target set comprises: (a) a polypeptide comprising a first target set nucleic acid binding domain and a first target, and (b) a first target set nucleic acid comprising a first target set polypeptide binding sequence, wherein the first target set polypeptide binding sequence is capable of binding to the first target set nucleic acid binding domain. In certain embodiments, the second target set comprises: (a) a polypeptide comprising a second target set nucleic acid binding domain and a second target, wherein the first target and the second target are different, and (b) a second target set nucleic acid comprising a second target set polypeptide binding sequence, wherein the second target set polypeptide binding sequence is capable of binding to the second target set nucleic acid binding domain. In certain embodiments, the probe set comprises: (a) a polypeptide comprising a probe set nucleic acid binding domain and a probe, and (b) a probe set nucleic acid comprising a probe set polypeptide binding sequence, wherein the probe set polypeptide binding sequence is capable of binding to the probe set nucleic acid binding domain. In certain embodiments, at least one of: a) both the first target set nucleic acid and the second target set nucleic acid comprise at least one addressable portion; and b) the probe set nucleic acid comprises at least one addressable portion. In certain embodiments, the reaction composition is incubated under reaction conditions such that a first binding complex may form; wherein the first binding complex comprises the first target set and the probe set; and wherein a second binding complex may form, wherein the second binding complex comprises the second target set and the probe set. In certain embodiments, a label associated with the first binding complexes that have been formed is detected using at least one of the at least one addressable portions and a label associated with the second binding complexes that have been formed is detected using at least one of the at least one addressable portions. In certain embodiments, the binding affinity of the first target to the probe is compared with the binding affinity of the second target to the probe by determining a ratio of first binding complexes formed to second binding complexes formed.

In certain embodiments, a method for detecting inhibition of binding complex formation by at least one potential inhibitor of binding complex formation is provided. In certain embodiments, a first reaction composition is formed comprising a target set and a probe set, and a second reaction composition is formed comprising the target set, the probe set, and at least one potential inhibitor of binding complex formation. In certain embodiments, the target set comprises: (a) a polypeptide comprising a target set nucleic acid binding domain and a target, and (b) a target set nucleic acid comprising a target set polypeptide binding sequence, wherein the target set polypeptide binding sequence is capable of binding to the target set nucleic acid binding domain. In certain embodiments, the probe set comprises: (a) a polypeptide comprising a probe set nucleic acid binding domain and a probe, and (b) a probe set nucleic acid comprising a probe set polypeptide binding sequence, wherein the probe set polypeptide binding sequence is capable of binding to the probe set nucleic acid binding domain. In certain embodiments, at least one of the first nucleic acid and the second nucleic acid comprises at least one addressable portion. In certain embodiments, incubating the first reaction composition is incubated under reaction conditions such that the first reaction composition forms a first test composition and incubating the second reaction composition under reaction conditions such that the second reaction composition forms a second test composition. In certain embodiments, if the probe interacts with the target in the first test composition, a binding complex is produced, wherein the binding complex comprises the probe set and the target set. In certain embodiments, if the probe interacts with the target in the second test composition, the binding complex is produced, wherein the binding complex comprises the probe set and the target set. In certain embodiments, a first detectable signal value is detected from a label associated with the binding complexes that have been formed in the first test composition, and a second detectable signal value is detected from a label associated with the binding complexes that have been formed in the second test composition. In certain embodiments, the detecting the first detectable signal value comprises using at least one of the at least one addressable portions and the detecting the second detectable signal value comprises using at least one of the at least one addressable portions. In certain embodiments, a threshold difference between the first detectable signal value and the second detectable signal value indicates inhibition of binding complex formation by the at least one potential inhibitor of binding complex formation.

### BRIEF DESCRIPTION OF THE FIGURES

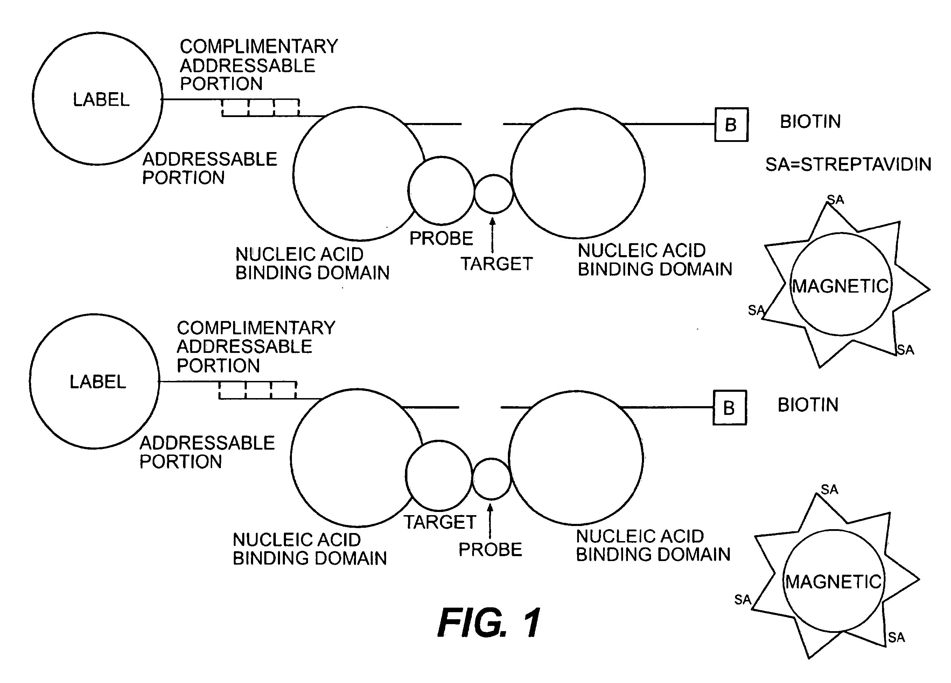

Figure 1 shows binding complexes according to certain embodiments.

### DETAILED DESCRIPTION OF CERTAIN EMBODIMENTS

In this application, the use of the singular includes the plural unless specifically stated otherwise. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise.
The section headings used herein are for organizational purposes only, and are not to be construed as limiting the subject matter described.

### Certain Definitions and Terms

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory or otherwise is naturally-occurring.

As used herein, "substantially pure" means an object species is the predominant species present (i.e., on a molar basis it is more abundant than any other individual species in the composition). In certain embodiments, a substantially purified fraction is a composition wherein the object species comprises at least about 50 percent (on a molar basis) of all macromolecular species present. In certain embodiments, a substantially pure composition will comprise more than about 80%, 85%, 90%, 95%, or 99% of all macromolar species present in the composition. In certain embodiments, the object species is purified to essential homogeneity (contaminant species cannot be detected in the composition by conventional detection methods) wherein the composition consists essentially of a single macromolecular species.

The terms "antagonist" or "inhibitor" refer to a molecule that reduces a biological activity of a biological molecule of interest. Inhibitors include, but are not limited to, molecules that completely block a biological activity as well as molecules that reduce a biological activity while not completely blocking that biological activity. The mechanism of action of the inhibitor or antagonist is not limited. Thus, an inhibitor may reduce the biological activity of a molecule by any mechanism, including, but not limited to, preventing or reducing binding of the molecule to a second molecule; preventing or reducing modification of the molecule (e.g., preventing or reducing phosphorylation of the molecule); and modifying the molecule. In certain embodiments, an inhibitor need not directly act on the biological molecule of interest, but may act on other molecules that regulate the activity of the biological molecule of interest.

The term "enhancer", when used to refer to an enhancer of an interaction between a target and a probe, refers to a molecule that increases the biological activity of a biological molecule of interest. The mechanism of action of an enhancer is not limited. Thus, an enhancer may increase the biological activity of a molecule by any mechanism, including, but not limited to, increasing the binding affinity between a target and a probe, and modifying a molecule where the modification results in increased biological activity. In certain embodiments, an enhancer need not directly act on the biological molecule of interest, but may act on other molecules that regulate the activity of the biological molecule of interest.

The term "operably linked" as used herein refers to components that are in a relationship permitting them to function in their intended manner. For example, a control sequence "operably linked" to a coding sequence is connected in such a way that expression of the coding sequence is achieved under conditions compatible with the control sequences.

The term "control sequence" as used herein refers to polynucleotide sequences which may effect the expression and processing of coding sequences to which they are connected. The nature of such control sequences may differ depending upon the host organism. According to certain embodiments, control sequences for prokaryotes may include promoter, ribosomal binding site, and transcription termination sequences. According to certain embodiments, control sequences for eukaryotes may include promoters and transcription termination sequences. In certain embodiments, "control sequences" can include leader sequences and/or fusion polypeptide sequences.

The terms "specifically interacts" and "specific interactions" refer to a first molecule that demonstrates an ability to specifically interact with one or more different molecules. While the first molecule may bind to molecules other than the one or more different molecules, the affinity of the first molecule for certain molecules other than the one or more different molecules is significantly lower than the affinity of the first molecule for the one or more different molecules. In certain embodiments, the first molecule may have greater affinity for two or more different molecules than certain other molecules. In certain such embodiments, the first molecule specifically interacts with each of the two or more different molecules. In certain embodiments, a first molecule is capable of specific interactions with a second molecule when the affinity of the first molecule for the second molecule is at least 10%, 30%, 50%, 70%, or 90% greater than the affinity of the first molecule for certain molecules other than the second molecule.

Certain embodiments comprise an interaction between a first molecule and at least one molecule of a molecule set, wherein the molecule set may be one type of molecule or more than one different type of molecule. In certain such embodiments, the first molecule specifically interacts with each molecule of a molecule set. While the first molecule may bind to molecules other than the molecules of the molecule set, the affinity of the first molecule for certain molecules other than the molecules of the molecule set is significantly lower than the affinity of the first molecule for each molecule of the molecule set. In certain embodiments, a first molecule is capable of specific interactions with each molecule of a molecule set when the affinity of the first molecule for each molecule of a molecule set is at least 10%, 30%, 50%, 70%, or 90% greater than the affinity of the first molecule for certain molecules other than the molecules of the molecule set.

The term "detecting", when used to refer to detecting a target, refers to determining the presence of a target. In certain embodiments, detecting a target does not require identification or characterization of the target.

The term "detectable signal value" refers to a value of the signal that is detected from a label. In certain embodiments, the detectable signal value is the amount or intensity of signal that is detected from a label. Thus, if there is no detectable signal from a label, its detectable signal value is zero (0). In certain embodiments, the detectable signal value is a characteristic of the signal other than the amount or intensity of the signal, such as the spectra, wavelength, color, or lifetime of the signal.

The term "detectably different signal value" means that one or more detectable signal values are distinguishable from one another by at least one detection method.

The term "threshold difference between detectable signal values" refers to a set difference between a first detectable signal value and a second detectable signal value that results when a potential inhibitor inhibits binding complex formation, but that does not result when a potential inhibitor does not inhibit binding complex formation.

As used herein, an "affinity set" is a set of molecules that specifically bind to one another. Exemplary affinity sets include, but are not limited to, biotin and avidin, biotin and streptavidin, His6 tag and nickel, receptor and ligand, antibody and ligand, antibody and antigen, a polynucleotide sequence and its complement, a polynucleotide and a polypeptide that specifically binds that polynucleotide, and affinity binding chemicals available from Prolinx™ (Bothell, WA) as exemplified, e.g., by U.S. Patent Nos. 5,831,046; 5,852,178; 5,859,210; 5,872,224; 5,877,297; 6,008,406; 6,013,783; 6,031,117; and 6,075,126. In certain embodiments, affinity sets that are bound may be unbound. For example, polynucleotide sequences that are hybridized may be denatured, and biotin bound to streptavidin may be heated and become unbound.

The term "nucleotide base", as used herein, refers to a substituted or unsubstituted aromatic ring or rings. In certain embodiments, the aromatic ring or rings contain at least one nitrogen atom. In certain embodiments, the nucleotide base is capable of forming Watson-Crick and/or Hoogsteen hydrogen bonds with an appropriately complementary nucleotide base. Exemplary nucleotide bases and analogs thereof include, but are not limited to, naturally occurring nucleotide bases adenine, guanine, cytosine, uracil, thymine, and analogs of the naturally occurring nucleotide bases, e.g., 7-deazaadenine, 7-deazaguanine, 7-deaza-8-azaguanine, 7-deaza-8-azaadenine, N6 -Δ2 -isopentenyladenine (6iA), N6 -Δ2 - isopentenyl-2-methylthioadenine (2ms6iA), N2 -dimethylguanine (dmG), 7-methylguanine (7mG), inosine, nebularine, 2-aminopurine, 2-amino-6-chloropurine, 2,6-diaminopurine, hypoxanthine, pseudouridine, pseudocytosine, pseudoisocytosine, 5-propynylcytosine, isocytosine, isoguanine, 7-deazaguanine, 2-thiopyrimidine, 6-thioguanine, 4-thiothymine, 4-thiouracil, *O*⁶-methylguanine, *N*⁶-methyladenine, *O*⁴-methylthymine, 5,6-dihydrothymine, 5,6-dihydrouracil, pyrazolo[3,4-D]pyrimidines (see, e.g., U.S. Patent Nos. 6,143,877 and 6,127,121 and PCT published application WO 01/38584), ethenoadenine, indoles such as nitroindole and 4-methylindole, and pyrroles such as nitropyrrole. Certain exemplary nucleotide bases can be found, e.g., in Fasman, 1989, Practical Handbook of Biochemistry and Molecular Biology, pp. 385-394, CRC Press, Boca Raton, Fla., and the references cited therein.

The term "nucleotide", as used herein, refers to a compound comprising a nucleotide base linked to the C-1' carbon of a sugar, such as ribose, arabinose, xylose, and pyranose, and sugar analogs thereof. The term nucleotide also encompasses nucleotide analogs. The sugar may be substituted or unsubstituted. Substituted ribose sugars include, but are not limited to, those riboses in which one or more of the carbon atoms, for example the 2'-carbon atom, is substituted with one or more of the same or different Cl, F, -R, -OR, - NR₂ or halogen groups, where each R is independently H, C₁-C₆ alkyl or C₅-C₁₄ aryl. Exemplary riboses include, but are not limited to, 2'-(C1 -C6)alkoxyribose, 2'-(C5 -C14)aryloxyribose, 2',3'-didehydroribose, 2'-deoxy-3'-haloribose, 2'-deoxy-3'-fluororibose, 2'-deoxy-3'-chlororibose, 2'-deoxy-3'-aminoribose, 2'-deoxy-3'-(C1 - C6)alkylribose, 2'-deoxy-3'-(C1 -C6)alkoxyribose and 2'-deoxy-3'-(C5 - C14)aryloxyribose, ribose, 2'-deoxyribose, 2',3'-dideoxyribose, 2'-haloribose, 2'-fluororibose, 2'-chlororibose, and 2'-alkylribose, e.g., 2'-O-methyl, 4'-α-anomeric nucleotides, 1'-α-anomeric nucleotides, 2'-4'- and 3'-4'-linked and other "locked" or "LNA", bicyclic sugar modifications (see, e.g., PCT published application nos. WO 98/22489, WO 98/39352;, and WO 99/14226). Exemplary LNA sugar analogs within a polynucleotide include, but are not limited to, the structures: 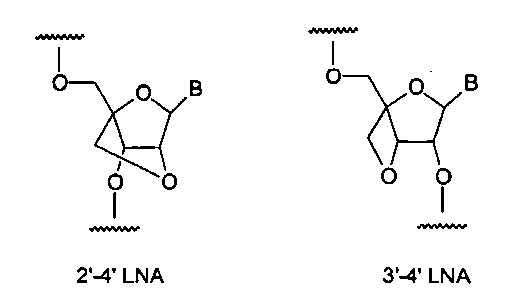 where B is any nucleotide base.

Modifications at the 2'- or 3'-position of ribose include, but are not limited to, hydrogen, hydroxy, methoxy, ethoxy, allyloxy, isopropoxy, butoxy, isobutoxy, methoxyethyl, alkoxy, phenoxy, azido, amino, alkylamino, fluoro, chloro and bromo. Nucleotides include, but are not limited to, the natural D optical isomer, as well as the L optical isomer forms (see, e.g., Garbesi (1993) Nucl. Acids Res. 21:4159-65; Fujimori (1990) J. Amer. Chem. Soc. 112:7435; Urata, (1993) Nucleic Acids Symposium Ser. No. 29:69-70). When the nucleotide base is purine, e.g. A or G, the ribose sugar is attached to the N⁹-position of the nucleotide base. When the nucleotide base is pyrimidine, e.g. C, T or U, the pentose sugar is attached to the N¹-position of the nucleotide base, except for pseudouridines, in which the pentose sugar is attached to the C5 position of the uracil nucleotide base (see, e.g., Kornberg and Baker, (1992) DNA Replication, 2nd Ed., Freeman, San Francisco, CA).

One or more of the pentose carbons of a nucleotide may be substituted with a phosphate ester having the formula:

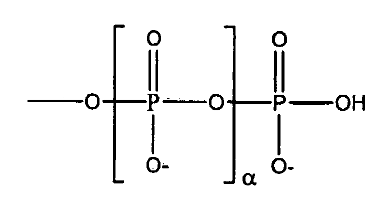

where a is an integer from 0 to 4. In certain embodiments, α is 2 and the phosphate ester is attached to the 3'- or 5'-carbon of the pentose. In certain embodiments, the nucleotides are those in which the nucleotide base is a purine, a 7-deazapurine, a pyrimidine, or an analog thereof. "Nucleotide 5'-triphosphate" refers to a nucleotide with a triphosphate ester group at the 5' position, and are sometimes denoted as "NTP", or "dNTP" and "ddNTP" to particularly point out the structural features of the ribose sugar. The triphosphate ester group may include sulfur substitutions for the various oxygens, e.g. α-thio-nucleotide 5'-triphosphates. For a review of nucleotide chemistry, see: Shabarova, Z. and Bogdanov, A. Advanced Organic Chemistry of Nucleic Acids, VCH, New York, 1994.

The term "nucleotide analog", as used herein, refers to embodiments in which the pentose sugar and/or the nucleotide base and/or one or more of the phosphate esters of a nucleotide may be replaced with its respective analog. In certain embodiments, exemplary pentose sugar analogs are those described above. In certain embodiments, the nucleotide analogs have a nucleotide base analog as described above. In certain embodiments, exemplary phosphate ester analogs include, but are not limited to, alkylphosphonates, methylphosphonates, phosphoramidates, phosphotriesters, phosphorothioates, phosphorodithioates, phosphoroselenoates, phosphorodiselenoates, phosphoroanilothioates, phosphoroanilidates, phosphoroamidates, boronophosphates, etc., and may include associated counterions.

Also included within the definition of "nucleotide analog" are nucleotide analog monomers which can be polymerized into polynucleotide analogs in which the DNA/RNA phosphate ester and/or sugar phosphate ester backbone is replaced with a different type of internucleotide linkage. Exemplary polynucleotide analogs include, but are not limited to, peptide nucleic acids, in which the sugar phosphate backbone of the polynucleotide is replaced by a peptide backbone.

As used herein, the terms "polynucleotide", "oligonucleotide", and "nucleic acid" are used interchangeably and mean single-stranded and double-stranded polymers of nucleotide monomers, including 2'-deoxyribonucleotides (DNA) and ribonucleotides (RNA) linked by internucleotide phosphodiester bond linkages, or internucleotide analogs, and associated counter ions, e.g., H⁺, NH₄⁺, trialkylammonium, Mg²⁺, Na⁺ and the like. A nucleic acid may be composed entirely of deoxyribonucleotides, entirely of ribonucleotides, or chimeric mixtures thereof. The nucleotide monomer units may comprise any of the nucleotides described herein, including, but not limited to, naturally occurring nucleotides and nucleotide analogs. Nucleic acids typically range in size from a few monomeric units, e.g. 5-40 when they are sometimes referred to in the art as oligonucleotides, to several thousands of monomeric nucleotide units. Unless denoted otherwise, whenever a nucleic acid sequence is represented, it will be understood that the nucleotides are in 5' to 3' order from left to right and that "A" denotes deoxyadenosine or an analog thereof, "C" denotes deoxycytidine or an analog thereof, "G" denotes deoxyguanosine or an analog thereof, and "T" denotes thymidine or an analog thereof, unless otherwise noted.

Nucleic acids include, but are not limited to, genomic DNA, cDNA, hnRNA, mRNA, rRNA, tRNA, fragmented nucleic acid, nucleic acid obtained from subcellular organelles such as mitochondria or chloroplasts, and nucleic acid obtained from microorganisms or DNA or RNA viruses that may be present on or in a biological sample.

Nucleic acids may be composed of a single type of sugar moiety, e.g., as in the case of RNA and DNA, or mixtures of different sugar moieties, e.g., as in the case of RNA/DNA chimeras. In certain embodiments, nucleic acids are ribopolynucleotides and 2'-deoxyribopolynucleotides according to the structural formulae below. 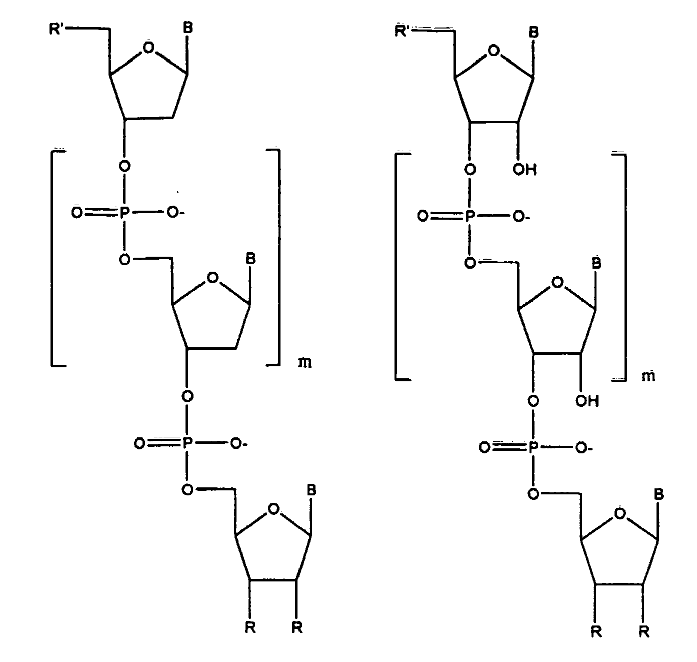 wherein each B is independently the base moiety of a nucleotides, e.g., a purine, a 7-deazapurine, a pyrimidine, or an analog nucleotide; each m defines the length of the respective nucleic acid and can range from zero to thousands, tens of thousands, or even more; each R is independently selected from the group comprising hydrogen, halogen, --R", --OR", and --NR"R", where each R" is independently (C1 -C6) alkyl or (C5 -C14) aryl, or two adjacent Rs are taken together to form a bond such that the ribose sugar is 2',3'-didehydroribose; and each R' is independently hydroxyl or 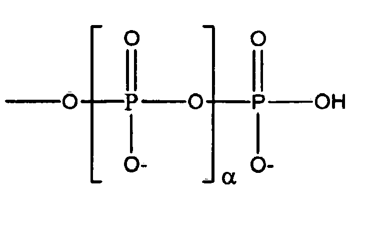 where a is zero, one or two.

In certain embodiments of the ribopolynucleotides and 2'-deoxyribopolynucleotides illustrated above, the nucleotide bases B are covalently attached to the C1' carbon of the sugar moiety as previously described.

The terms "nucleic acid", "polynucleotide", and "oligonucleotide" may also include nucleic acid analogs, polynucleotide analogs, and oligonucleotide analogs. The terms "nucleic acid analog", "polynucleotide analog" and "oligonucleotide analog" are used interchangeably and, as used herein, refer to a nucleic acid that contains at least one nucleotide analog and/or at least one phosphate ester analog and/or at least one pentose sugar analog. Also included within the definition of nucleic acid analogs are nucleic acids in which the phosphate ester and/or sugar phosphate ester linkages are replaced with other types of linkages, such as N-(2-aminoethyl)-glycine amides and other amides (see, e.g., Nielsen et al., 1991, Science 254: 1497-1500; WO 92/20702; U.S. Pat. No. 5,719,262; U.S. Pat. No. 5,698,685;); morpholinos (see, e.g., U.S. Pat. No. 5,698,685; U.S. Pat. No. 5,378,841; U.S. Pat. No. 5,185,144); carbamates (see, e.g., Stirchak & Summerton, 1987, J. Org. Chem. 52: 4202); methylene(methylimino) (see, e.g., Vasseur et al., 1992, J. Am. Chem. Soc. 114: 4006); 3'-thioformacetals (see, e.g., Jones et al., 1993, J. Org. Chem. 58: 2983); sulfamates (see, e.g., U.S. Pat. No. 5,470,967); 2-aminoethylglycine, commonly referred to as PNA (see, e.g., Buchardt, WO 92/20702; Nielsen (1991) Science 254:1497-1500); and others (see, e.g., U.S. Pat. No. 5,817,781; Frier & Altman, 1997, Nucl. Acids Res. 25:4429 and the references cited therein). Phosphate ester analogs include, but are not limited to, (i) C₁-C₄ alkylphosphonate, e.g. methylphosphonate; (ii) phosphoramidate; (iii) C₁-C₆ alkyl-phosphotriester; (iv) phosphorothioate; and (v) phosphorodithioate.

The terms "annealing" and "hybridization" are used interchangeably and mean the base-pairing interaction of one nucleic acid with another nucleic acid that results in formation of a duplex, triplex, or other higher-ordered structure. In certain embodiments, the primary interaction is base specific, e.g., A/T and G/C, by Watson/Crick and Hoogsteen-type hydrogen bonding. In certain embodiments, base-stacking and hydrophobic interactions may also contribute to duplex stability.

The term "polypeptide binding sequence" refers to a nucleic acid sequence that binds a polypeptide. In certain embodiments, a single polypeptide binding sequence binds one or more polypeptides. In certain such embodiments, multiple different polypeptides may bind to a polypeptide binding sequence at different times such that only one polypeptide binds to the polypeptide binding sequence at any one time. In certain embodiments, multiple polypeptides bind to the polypeptide binding sequence at the same time. In certain such embodiments, the polypeptides may be identical (e.g., homodimers) or the polypeptides may be different (e.g., heterodimers).

In certain embodiments, a polypeptide binding sequence may be a naturally occurring polypeptide binding sequence or, may be a non-naturally occurring polypeptide binding sequence. In certain embodiments, a polypeptide binding sequence is designed based on known polypeptide binding sequences. In certain embodiments, one or more polypeptide binding sequences are selected from a library of randomly generated nucleic acid sequences.

In certain embodiments, polypeptide binding sequences may comprise RNA sequences (both single-stranded and double-stranded) or DNA sequences (both single-stranded and double-stranded).

Many non-limiting exemplary nucleic acid sequences that bind polypeptides are known to those skilled in the art. For example without limitation, certain nucleic acid sequences that bind polypeptides include, but are not limited to, enhancers, activating sequences, response elements, origins of replication, promoters, and other sequences.

The term "polypeptide" is used herein as a generic term to refer to any polypeptide comprising two or more amino acids joined to each other by peptide bonds or modified peptide bonds. The term "polypeptide" encompasses polypeptides regardless of length or origin, comprising molecules that are recombinantly produced or naturally occurring, full length or truncated, having a natural sequence or mutated sequence, with or without post-translational modification, whether produced in mammalian cells, bacterial cells, or any other expression system. In certain embodiments, polypeptides are randomly generated by any methods, including, but not limited to, methods discussed herein. In certain embodiments, shorter polypeptides are derived by digestion of larger polypeptides. In certain embodiments, polypeptides fold into a three dimensional shape determined partially by the amino acid sequence of the polypeptide.

In certain embodiments, polypeptides comprise domains. The term "domain" refers to at least a portion of a polypeptide sequence. In certain embodiments, a polypeptide may comprise one or more domains. In certain embodiments, a domain comprises a continuous stretch of amino acids able to fold into a three dimensional shape independent of the rest of the polypeptide. In certain embodiments, certain functions are associated with domains. A nonlimiting example is a "DNA binding domain" that comprises a domain that binds to DNA under appropriate conditions. In certain embodiments, a portion of a polypeptide is referred to as a domain regardless of whether a function is associated with that portion.

In certain embodiments, where a function is associated with a particular domain, that domain is used in a fusion polypeptide to give the fusion polypeptide the particular function associated with the domain. For example and not limitation, where a DNA binding domain is associated with the function of binding to a specific DNA sequence, then that DNA binding domain may be fused to a heterologous polypeptide to generate a fusion polypeptide that will bind to that same DNA sequence. In certain embodiments, two or more domains may be fused to create a fusion polypeptide with multiple functions. In certain embodiments, fusion polypeptides comprise a polypeptide with one or more domains added and/or removed, which imparts on the fusion polypeptide one or more specific functions.

The term "randomized", as used to refer to polypeptide sequences, encompasses fully random sequences (for example, but not limited to, sequences selected by phage display methods) and encompasses sequences in which at least one residue of a naturally occurring molecule is replaced by an amino acid residue not appearing in that position in the naturally occurring molecule. In certain embodiments, certain specific polypeptide sequences of interest are identified from a library of random polypeptide sequences. Exemplary methods for identifying polypeptide sequences of interest from a library of random polypeptide sequences include, but are not limited to, phage display, *E*. *coli* display, ribosome display, yeast-based screening, RNA-peptide screening, chemical screening, rational design, polypeptide structural analysis, and the like. Certain exemplary randomized polypeptides and certain exemplary methods of generating them appear in, e.g., PCT Published Application No. WO 00/24782, published May 4, 2000.

As used herein, the twenty conventional amino acids and their abbreviations follow conventional usage. Stereoisomers (e.g., D-amino acids) of the twenty conventional amino acids, unnatural amino acids, and other unconventional amino acids may also be suitable components for polypeptides. In the polypeptide notation used herein, the left-hand direction is the amino terminal direction and the right-hand direction is the carboxy-terminal direction, in accordance with standard usage and convention.

The term "variant" as used herein refers to any alteration of a polypeptide, including, but not limited to, changes in amino acid sequence, substitutions of one or more amino acids, addition of one or more amino acids, deletion of one or more amino acids, and alterations to the amino acids themselves.

In certain embodiments, variants of polypeptides involve conservative amino acid substitutions. Conservative amino acid substitution may involve replacing one amino acid with another that has, e.g., similar hydrophobicity, hydrophilicity, charge, or aromaticity. In certain embodiments, conservative amino acid substitutions are made on the basis of similar hydropathic indices. A hydropathic index takes into account the hydrophobicity and charge characteristics of an amino acid, and in certain embodiments, may be used as a guide for selecting conservative amino acid substitutions. The hydropathic index is discussed, e.g., in Kyte et al., J. Mol. Biol., 157:105-131 (1982). In certain embodiments, conservative amino acid substitutions may be made on the basis of any of the aforementioned characteristics.

In certain embodiments, polypeptide sequences are created from conservative and/or non-conservative modifications of the amino acid sequences of certain native molecules.

In certain embodiments, conservative modifications produce molecules having functional and chemical characteristics similar to those of the molecule from which such modifications are made. In contrast, in certain embodiments, substantial modifications in the functional and/or chemical characteristics of the molecules may be accomplished by selecting substitutions in the amino acid sequence that differ significantly in their effect on maintaining (a) the structure of the molecular backbone in the area of the substitution, for example, as a sheet or helical conformation, (b) the charge or hydrophobicity of the molecule at the target site, or (c) the size of the molecule.

A skilled artisan will be able to determine suitable variants of the polypeptide as set forth herein using well-known techniques. In certain embodiments, one skilled in the art may identify suitable areas of the molecule that may be changed without destroying activity by targeting regions not believed to be important for activity. In certain embodiments, one can identify residues and portions of the molecules that are conserved among similar polypeptides. In certain embodiments, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, one can predict the importance of amino acid residues in a polypeptide that correspond to amino acid residues which are important for activity or structure in similar polypeptides. One skilled in the art may opt for chemically similar amino acid substitutions for such predicted important amino acid residues.

One skilled in the art can also analyze the three-dimensional structure and amino acid sequence in relation to that structure in similar polypeptides. In view of such information, one skilled in the art may predict the alignment of amino acid residues of a polypeptide with respect to its three dimensional structure. In certain embodiments, one skilled in the art may choose not to make radical changes to amino acid residues predicted to be on the surface of the polypeptide, since such residues may be involved in important interactions with other molecules. Moreover, one skilled in the art may generate test variants containing a single amino acid substitution at each desired amino acid residue. The variants can then be screened using activity assays known to those skilled in the art. Such variants could be used to gather information about suitable variants. For example, if one discovered that a change to a particular amino acid residue resulted in destroyed, undesirably reduced, or unsuitable activity, variants with such a change may be avoided. In other words, based on information gathered from such routine experiments, one skilled in the art can readily determine the amino acids where further substitutions should be avoided either alone or in combination with other mutations.

A number of scientific publications have been devoted to the prediction of secondary structure. See Moult J., Curr. Op. in Biotech., 7(4):422-427 (1996), Chou et al., Biochemistry, 13(2):222-245 (1974); Chou et al., Biochemistry, 113(2):211-222 (1974); Chou et al., Adv. Enzymol. Relat. Areas Mol. Biol., 47:45-148 (1978); Chou et al., Ann. Rev. Biochem., 47:251-276 and Chou et al., Biophys. J., 26:367-384 (1979). Moreover, computer programs are currently available to assist with predicting secondary structure. One method of predicting secondary structure is based upon homology modeling. For example, two polypeptides or polypeptides which have a sequence identity of greater than 30%, or similarity greater than 40% often have similar structural topologies. The recent growth of the protein structural database (PDB) has provided enhanced predictability of secondary structure, including the potential number of folds within a polypeptide's or protein's structure. See Holm et al., Nucl. Acid. Res., 27(1):244-247 (1999). It has been suggested (Brenner et al., Curr. Op. Struct. Biol., 7(3):369-376 (1997)) that there are a limited number of folds in a given polypeptide and that once a critical number of structures have been resolved, structural prediction will become dramatically more accurate.

Additional methods of predicting secondary structure include "threading" (Jones, D., Curr. Opin. Struct. Biol., 7(3):377-87 (1997); Sippl et al., Structure, 4(1):15-19 (1996)), "profile analysis" (Bowie et al., Science, 253:164-170 (1991); Gribskov et al., Meth. Enzym., 183:146-159 (1990); Gribskov et al., Proc. Nat. Acad. Sci., 84(13):4355-4358 (1987)), and "evolutionary linkage" (See Holm, *supra* (1999), and Brenner, *supra* (1997)).

Alterations to the amino acids may include, but are not limited to, glycosylation, methylation, phosphorylation, biotinylation, and any covalent and noncovalent additions to a polypeptide that do not result in a change in amino acid sequence. "Amino acid" as used herein refers to any amino acid, natural or nonnatural, that may be incorporated, either enzymatically or synthetically, into a polypeptide.

According to certain embodiments, amino acid substitutions are those which: (1) reduce susceptibility to proteolysis, (2) reduce susceptibility to oxidation, (3) alter binding affinity for forming polypeptide complexes, (4) alter binding affinities, or (5) confer or modify other physicochemical or functional properties on such polypeptides. According to certain embodiments, single or multiple amino acid substitutions (in certain embodiments, conservative amino acid substitutions) may be made in the naturally-occurring sequence (in certain embodiments, in the portion of the polypeptide outside the domain(s) forming intermolecular contacts). In certain embodiments, a conservative amino acid substitution typically may not substantially change the structural characteristics of the parent sequence (e.g., a replacement amino acid should not tend to break a helix that occurs in the parent sequence, or disrupt other types of secondary structure that characterizes the parent sequence). Examples of art-recognized polypeptide secondary and tertiary structures are described, e.g., in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et al. Nature 354:105 (1991).

The term "polypeptide-polypeptide interaction" refers to any direct or indirect interaction between two or more polypeptides. Polypeptide-polypeptide interactions include, but are not limited to, situations where polypeptides contact each other. Polypeptide-polypeptide interactions also include, but are not limited to, situations where there is no physical contact between the interacting polypeptides. Polypeptide-polypeptide interactions include, but are not limited to, association of polypeptides, binding of polypeptides to other polypeptides, and chemical and conformational modification of polypeptides by other polypeptides. In certain embodiments, polypeptide-polypeptide interactions may be mediated by non-covalent bonds formed between the polypeptides. In certain embodiments, polypeptide-polypeptide interactions may be mediated by auxiliary molecules, which may act as a bridge between the interacting polypeptides. Examples of processes involving polypeptide-polypeptide interactions include, but are not limited to, transcriptional activation or repression, antigen binding to antibody, ligand binding to receptor, a viral polypeptide binding to a polypeptide on a cell surface, forms of signal transduction (e.g., phosphorylation of polypeptides by polypeptide kinases), and the formation of dimers and multimers.

The term "dimer" as applied to polypeptides or molecules comprising polypeptides refers to molecules having two polypeptides associated covalently or non-covalently. The term "homodimer" refers to a dimer wherein the two polypeptides are the same. The term "heterodimer" refers to a dimer wherein the two polypeptides are different.

The term "multimer" as applied to polypeptides or molecules comprising polypeptides refers to molecules having two or more polypeptide chains associated covalently, non-covalently, or by both covalent and non-covalent interactions.

The term "complex" refers to two or more molecules associated covalently, non-covalently, or by both covalent and non-covalent interactions. Components of a complex may include, but are not limited to, polypeptides, chemical ligands, nucleic acids, and other molecules.

The term "nucleic acid binding domain" refers to a domain that binds specifically or non-specifically to a nucleic acid. In certain embodiments, a particular nucleic acid binding domain binds specifically to a particular nucleic acid sequence.

In certain embodiments, a particular nucleic acid binding domain binds specifically to a particular type of nucleic acid. Examples of types of nucleic acids that may be bound by nucleic acid binding domains include, but are not limited to, RNA (both single stranded and double stranded) and DNA (both single stranded and double stranded). In certain embodiments, nucleic acid binding domains bind to particular sequences of one type of nucleic acid, but will not bind to similar or identical sequences of another type of nucleic acid. For example without limitation, a particular nucleic acid binding domain may bind to a particular DNA sequence, but will not bind to a similar sequence in an RNA.

In certain embodiments, nucleic acid binding domains may function as dimers or multimers. In certain embodiments, such nucleic acid binding domains may specifically bind to a nucleic acid with greater affinity as part of a dimer or multimer than as a monomer. In certain embodiments, a nucleic acid binding domain that binds to a particular sequence as a dimer or multimer may not bind to the nucleic acid as a monomer. In certain embodiments, dimers or multimers of nucleic acid binding domains may comprise two or more different nucleic acid binding domains (e.g., in the case of a dimer, the dimer may be a heterodimer of two different nucleic acid binding domains). In certain embodiments, a nucleic acid binding domain may bind to a first nucleic acid as part of a homodimer and a second nucleic acid as part of a heterodimer. In certain such embodiments, the specificity of a first nucleic acid binding domain for a particular nucleic acid sequence may be provided by a second nucleic acid binding domain with which the first nucleic acid binding domain forms a heterodimer.

In certain embodiments, a nucleic acid binding domain may be derived from a naturally occurring polypeptide. In certain embodiments, a nucleic acid binding domain may be non-natural. In certain embodiments, non-natural nucleic acid binding domains are created through mutagenesis of a nucleic acid that encodes a naturally occurring polypeptide, or other techniques known in the art. Certain methods for generating nucleic acid binding domains which can selectively bind to a specific nucleic acid sequence are known in the art. See e.g., U.S. Patent No. 5,198,346.

Certain nucleic acid binding domains are known in the art. Exemplary nucleic acid binding domains include, but are not limited to, the nucleic acid binding domains of p53, Jun, Fos, GCN4, GAL4, RAP1, and LexA.

As used herein, the term "fusion polypeptide" refers to a polypeptide joined to a heterologous polypeptide. In certain embodiments, a fusion polypeptide comprises both naturally occurring and non-natural amino acids. In certain embodiments, a fusion polypeptide comprises a bifunctional molecule. For example, in certain embodiments, a fusion polypeptide comprises a target fused to a nucleic acid binding domain. In certain embodiments, a fusion polypeptide comprises a probe fused to a nucleic acid binding domain. In certain embodiments, a fusion polypeptide comprises three or more different domains. In certain such embodiments, the three or more different domains are derived from three or more different sources. In certain embodiments, a polypeptide linker separates different domains of a fusion polypeptide.

In certain embodiments, a first polypeptide interacts with a second polypeptide through interactions with a third molecule that acts as a bridge. Certain examples of bridging molecules include, but are not limited to, ligands, small chemical molecules and other polypeptides.

### Certain Expression Methods

Various biological or chemical methods for producing a polypeptide are available.

In certain embodiments, biological methods are used for producing a polypeptide. In certain embodiments, standard recombinant DNA techniques are useful for the production of a polypeptide. Certain exemplary expression vectors, host cells, and methods for recovery of the expressed product are described below.

In certain embodiments, recombinant DNA techniques may be used to create a DNA encoding a polypeptide. In certain embodiments, the DNA encoding the polypeptide will be fused in frame to create a contiguous amino acid sequence containing sequences from at least two different sources. For example and not limitation, a DNA encoding a target may be fused to a DNA encoding a nucleic acid binding domain such that a fusion polypeptide comprising both domains results from expression of the DNA. Certain methods for cloning and engineering a DNA encoding a fusion polypeptide are known in the art.

In certain embodiments, a nucleic acid molecule encoding a polypeptide is inserted into an appropriate expression vector using standard ligation techniques. In certain embodiments, the vector may be selected to be functional in the particular host cell employed (i.e., the vector is compatible with the host cell machinery such that amplification of the gene and/or expression of the gene can occur). In certain embodiments, a nucleic acid molecule encoding a polypeptide may be amplified in prokaryotic, yeast, insect (e.g., baculovirus systems), or eukaryotic host cells. In certain embodiments, a nucleic acid molecule encoding a polypeptide may be expressed in prokaryotic, yeast, insect, or eukaryotic host cells. In certain embodiments, selection of the host cell will take into account, in part, whether a polypeptide is to be post-translationally modified (e.g., glycosylated and/or phosphorylated). In certain embodiments, yeast, insect, or mammalian host cells are selected to facilitate post-translational modifications. For a review of expression vectors, see, e.g., Meth. Enz. v. 185, (D.V. Goeddel, ed.), Academic Press Inc., San Diego, CA (1990).

In certain embodiments, expression vectors used in host cells will contain one or more of the following components: a promoter, one or more enhancer sequences, an origin of replication, a transcriptional termination sequence, a complete intron sequence containing a donor and acceptor splice site, a leader sequence for secretion, a ribosome binding site, a polyadenylation sequence, a polylinker region for inserting the nucleic acid encoding the polypeptide to be expressed, and/or a selectable marker element. Certain such sequences are discussed in more detail below.

Exemplary vector components include, but are not limited to, homologous (i.e., from the same species and/or strain as the host cell), heterologous (i.e., from a species other than the host cell species or strain), hybrid (i.e., a combination of different sequences from more than one source), synthetic, or native sequences which normally function to regulate gene expression. In various embodiments, a source of vector components may be any prokaryotic or eukaryotic organism, any vertebrate or invertebrate organism, or any plant, provided that the components are functional in, and can be activated by, the host cell machinery.

In certain embodiments, a leader or signal sequence may be used to direct secretion of a polypeptide. In certain embodiments, a signal sequence may be positioned within or directly at the 5' end of a polypeptide coding region. Certain signal sequences have been identified and, in certain embodiments, may be selected based upon the host cell used for expression. In certain embodiments, a signal sequence may be homologous (naturally occurring) or heterologous to a nucleic acid encoding a fusion polypeptide. In certain embodiments, a heterologous signal sequence selected should be one that is recognized and processed, i.e., cleaved by a signal peptidase, by the host cell. In certain embodiments involving prokaryotic host cells that do not recognize and process a homologous signal sequence, the signal sequence may be substituted with a prokaryotic signal sequence selected, e.g., from alkaline phosphatase, penicillinase, or heat-stable enterotoxin II leaders. In certain embodiments, for yeast secretion, a homologous signal sequence may be substituted by a yeast leader sequence. Exemplary yeast leader sequences include, but are not limited to, yeast invertase, alpha factor, and acid phosphatase leaders. In certain embodiments, mammalian cell expression using the homologous signal sequence may be satisfactory. In certain embodiments, other mammalian signal sequences may be suitable.

In certain embodiments, secretion of a polypeptide from a host cell will result in the removal of the signal peptide from the polypeptide. Thus, in such embodiments, the mature polypeptide will lack any leader or signal sequence.

In certain embodiments, such as where glycosylation is desired in a eukaryotic host cell expression system, one may manipulate the various presequences to improve glycosylation or yield. In certain embodiments, one may alter the peptidase cleavage site of a particular signal peptide, or add prosequences, which also may affect glycosylation. In certain embodiments, the final polypeptide product may have, in the -1 position (relative to the first amino acid of the mature polypeptide) one or more additional amino acids incident to expression, which may not have been totally removed. In certain embodiments, the final polypeptide product may have one or two amino acids found in the peptidase cleavage site, attached to the N-terminus. In certain embodiments, use of some enzyme cleavage sites may result in a slightly truncated form of the desired polypeptide, if the enzyme cuts at such area within the mature polypeptide.

In certain embodiments, the expression vectors may contain a promoter that is recognized by the host organism and operably linked to a nucleic acid molecule encoding a polypeptide. In certain embodiments, a native or heterologous promoter may be used depending on the host cell used for expression and the yield of polypeptide desired.

In certain embodiments, an enhancer sequence may be inserted into the vector to increase transcription in eukaryotic host cells. In certain embodiments, an enhancer may be spliced into the vector at a position 5' or 3' to the polypeptide coding region. In certain embodiments, the enhancer is located at a site 5' from the promoter.

In certain embodiments, vectors are those which are compatible with at least one host cell selected from bacterial, insect, and mammalian host cells. Exemplary vectors include, but are not limited to, cosmids, plasmids and modified viruses compatible with the selected host cell. In various embodiments, recombinant molecules may be introduced into host cells via transformation, transfection, infection, electroporation, or other techniques.

Exemplary host cells include, but are not limited to, prokaryotic host cells (such as E. coli) or eukaryotic host cells (such as a yeast cell, an insect cell, or a vertebrate cell). In certain embodiments, prokaryotic host cells such as E. coli produce unglycosylated polypeptide, which may possess advantages over the glycosylated eukaryotic molecules. In certain embodiments, the host cell, when cultured under appropriate conditions, expresses a polypeptide which can subsequently be collected from the culture medium (if the host cell secretes it into the medium). In certain embodiments, the host cell, when cultured under appropriate conditions, expresses a polypeptide which can be collected directly from the host cell producing it (if it is not secreted). In certain embodiments, selection of an appropriate host cell will take into account various factors, such as desired expression levels, polypeptide modifications that are desirable or necessary for activity, such as glycosylation or phosphorylation, and/or ease of folding into a biologically active molecule.

Certain suitable host cells are known in the art and many are available from the American Type Culture Collection (ATCC), Manassas, VA. Exemplary host cells include, but are not limited to, mammalian cells. Certain suitable mammalian host cells and methods for transformation, culture, amplification, screening and product production and purification are known in the art.

In certain embodiments, the host cells may be bacterial cells.

Many strains of yeast cells known to those skilled in the art are also available as host cells for expression of polypeptides in certain embodiments. In certain embodiments, the host cell may be Saccharomyces cerivisae.

In certain embodiments, insect cell systems may be used for the expression of polypeptides.

In certain embodiments, transformation or transfection of a nucleic acid molecule encoding a polypeptide into a selected host cell may be accomplished by known methods including methods such as calcium chloride, electroporation, microinjection, lipofection, or the DEAE-dextran method. In certain embodiments, the method selected will in part be a function of the type of host cell to be used. Certain methods are well known to the skilled artisan, and are set forth, for example, in Sambrook et al. Molecular Cloning: A Laboratory Manual (2d ed., Cold Spring Harbor Laboratory Press, Cold Spring Harbor, N.Y. (1989)).

In certain embodiments, the amount of a polypeptide produced by a host cell can be evaluated using standard methods known in the art. Exemplary methods include, but are not limited to, Western blot analysis, SDS-polyacrylamide gel electrophoresis, non-denaturing gel electrophoresis, HPLC separation, immunoprecipitation, and activity assays.

In various embodiments, purification of a polypeptide that has been secreted into the cell media may be accomplished using a variety of techniques. Exemplary techniques include, but are not limited to, affinity, immunoaffinity or ion exchange chromatography; molecular sieve chromatography; preparative gel electrophoresis or isoelectric focusing; chromatofocusing; and high pressure liquid chromatography. In certain embodiments, modified forms of a polypeptide may be prepared with affinity tags, such as hexahistidine or other small peptides such as FLAG (Eastman Kodak Co., New Haven, CT) or myc (Invitrogen) at either the carboxyl or amino terminus and purified by a one-step affinity column. In certain embodiments, polyhistidine binds with great affinity and specificity to nickel, thus an affinity column of nickel (such as the Qiagen® nickel columns) can be used for purification of polyhistidine-tagged fusion polypeptides. See for example, Ausubel et al., eds. (1993), Current Protocols in Molecular Biology, Section 10.11.8, John Wiley & Sons, New York. In certain embodiments, more than one purification step may be used.

In certain embodiments, polypeptides which are expressed in procaryotic host cells may be present in soluble form either in the periplasmic space or in the cytoplasm or in an insoluble form as part of intracellular inclusion bodies. In various embodiments, polypeptides can be extracted from the host cell using any standard technique known to the skilled artisan. In certain embodiments, the host cells can be lysed to release the contents of the periplasm/cytoplasm by French press, homogenization, and/or sonication followed by centrifugation.

In certain embodiments, soluble forms of a polypeptide present either in the cytoplasm or released from the periplasmic space may be further purified using methods known in the art. In certain embodiments, polypeptides are released from the bacterial periplasmic space by osmotic shock techniques.

If a polypeptide has formed inclusion bodies, they may often bind to the inner and/or outer cellular membranes and thus will be found primarily in the pellet material after centrifugation. In various embodiments, the inclusion bodies found in the pellet material may then be treated with one or more treatments or conditions to release, break apart, and/or solubilize the inclusion bodies. Exemplary treatments and conditions for releasing, breaking apart, and/or solubilizing the inclusion bodies include, but are not limited to, conditions that comprise pH extremes and treatment with one or more chaotropic partner. Examples of treatment with a chaotropic partner include, but are not limited to, treatment with a detergent, treatment with guanidine, treatment with guanidine derivatives, treatment with urea, and treatment with urea derivatives in the presence of a reducing partner such as dithiothreitol at alkaline pH or tris carboxyethyl phosphine at acid pH. In certain embodiments, the soluble polypeptide may then be analyzed using gel electrophoresis, immunoprecipitation, or the like. In certain embodiments, a solubilized polypeptide may be isolated using standard methods known in the art. See, e.g., Marston et al. (1990), Meth. Enz., 182: 264-75.

In certain embodiments, a polypeptide may not be biologically active upon isolation. In certain embodiments, methods known in the art for "refolding" or converting the polypeptide to its tertiary structure and generating disulfide linkages, may be used to restore biological activity. In certain embodiments, the biological activity may be restored by exposing the solubilized polypeptide to a pH usually above 7 in the presence of a particular concentration of a chaotrope.

In certain embodiments, polypeptides may be prepared by chemical synthesis methods. In certain embodiments, the chemical synthesis method may incorporate solid phase peptide synthesis. In certain embodiments, the chemical synthesis methods may use techniques known in the art. See, e.g., Merrifield et al. (1963), J. Am. Chem. Soc., 85: 2149; Houghten et al. (1985), Proc Natl Acad. Sci. USA, 82: 5132; and Stewart and Young (1984), Solid Phase Peptide Synthesis, Pierce Chemical Co., Rockford, IL. In certain embodiments, polypeptides may be synthesized with or without a methionine on the amino terminus. In certain embodiments, chemically synthesized polypeptides may be oxidized using methods known in the art to form disulfide bridges. In certain embodiments, polypeptides so prepared will retain at least one biological activity associated with a native or recombinantly produced polypeptide.

### Certain Exemplary Targets

The term "target" refers to any material tested with a probe wherein the test determines whether the probe specifically interacts with the target. Exemplary specific interactions include, but are not limited to, specific binding of one polypeptide to another polypeptide, specific binding of a polypeptide to a molecule, hybridization between nucleic acid molecules, interactions between antibodies and antigens, interactions between ligands and receptors, and interactions between aptamers and polypeptides. In certain embodiments, targets are materials that are tested with a probe, but the probe fails to specifically interact with them. In certain embodiments, targets are naturally occurring molecules. In certain embodiments, targets are synthetic molecules.

Exemplary targets include, but are not limited to, full length, or portions of, transcription factors, ligands, viral proteins, enzymes, proteins involved in signal transduction, cytoskeletal proteins, and other proteins. Exemplary targets include, but are not limited to, polypeptides. Exemplary targets include, but are not limited to, proteins expressed from different alleles (similar polypeptides with slightly different amino acids) and different polypeptide conformations (similar polypeptides with different secondary and tertiary structures). In certain embodiments, a target is chemically modified. Exemplary chemical modifications include, but are not limited to, modification by phosphorylation, methylation, and glycosylation.

Exemplary naturally occurring targets include, but are not limited to, signal molecules, such as hormones and other steroid-type molecules.

Exemplary targets include, but are not limited to, synthetic polypeptides, members of affinity sets, pharmaceuticals, and other organic small molecules.

In certain embodiments, targets comprise nucleic acids. In certain embodiments, a target nucleic acid may comprise RNA or DNA. Exemplary RNA targets include, but are not limited to, mRNA, rRNA, tRNA, viral RNA, and variants thereof. Exemplary variants of RNAs include, but are not limited to, splice variants, other RNA sequence variants, and RNA with chemical modifications. Exemplary DNA targets include, but are not limited to, genomic DNA, plasmid DNA, phage DNA, nucleolar DNA, mitochondrial DNA, and chloroplast DNA. Exemplary nucleic acids include, but are not limited to, cDNA, yeast artificial chromosomes (YAC's), bacterial artificial chromosomes (BAC's), other extrachromosomal DNA, and nucleic acid analogs. Exemplary nucleic acid analogs include, but are not limited to, LNAs, PNAs, PPG's, and other nucleic acid analogs.

In certain embodiments, targets comprise nucleic acid aptamers.

The person of ordinary skill will appreciate that while a target nucleic acid is typically described as a single-stranded molecule, the opposing strand of a double-stranded molecule comprises a complementary sequence that may also be used as a target.

In certain embodiments, targets are attached to polypeptides and tested for interactions with a probe. Exemplary targets that may be attached to a polypeptide include, but are not limited to, polypeptides (and variants thereof), nucleic acids, chemicals, and other molecules. In certain embodiments, where a polypeptide target is attached to another polypeptide, the polypeptide target comprises a randomly generated amino acid sequence.

In certain embodiments, targets are part of a target set. The term "target set" refers to a set of molecules comprising at least a target. In certain embodiments, each molecule of a target set may specifically interact with at least one other molecule of the target set, which, under the proper conditions, may allow for the assembly of a complex of at least some molecules of the target set. In certain embodiments, a target set comprises (1) a polypeptide comprising a target set nucleic acid binding domain and a target, and (2) a target set nucleic acid comprising a target set polypeptide binding sequence, wherein the target set polypeptide binding sequence is capable of binding to the target set nucleic acid binding domain. In certain embodiments, a target set may additionally comprise at least one additional molecule selected from at least one label, at least one separating moiety, and at least one addressable portion.

In certain embodiments, a target set comprises (1) a polypeptide comprising a target set nucleic acid binding domain and a target, (2) a target set nucleic acid comprising a target set polypeptide binding sequence, wherein the target set polypeptide binding sequence is capable of binding to the target set nucleic acid binding domain, and a complementary addressable portion and (3) a label covalently linked to an addressable portion. Under the appropriate reaction conditions, the nucleic acid binding domain binds to the polypeptide binding sequence and the addressable portion hybridizes to the complementary addressable portion. Under certain such conditions, the target is attached to the label through the target set nucleic acid. In certain embodiments, a probe specifically interacts with a target such that the probe is attached to the label through the target and the target set nucleic acid. In certain such embodiments, the specific interaction between the probe and the target is detected by detecting the label attached to the probe.

The term "target group" refers to a group of one or more target sets wherein each target set of the target group comprises at least one component common to all target sets of the target group. In certain embodiments, the target group may comprise a plurality of target sets that comprise the same target set nucleic acid binding domain and the same target set nucleic acid and comprise different targets. In certain embodiments, the target group may comprise some target sets with different targets and some target sets with the same target.

### Certain Exemplary Probes

The term "probe" or "target-specific probe" is any moiety that comprises a portion that may specifically interact with a target. Exemplary specific interactions include, but are not limited to, specific binding of one polypeptide to another polypeptide, specific binding of a polypeptide to a molecule, hybridization between nucleic acid molecules, interactions between antibodies and antigens, interactions between ligands and receptors, and interactions between aptamers and polypeptides. In certain embodiments, probes are naturally occurring molecules. In certain embodiments, probes are synthetic molecules.

Exemplary probes include, but are not limited to, full length, or portions of, transcription factors, ligands, viral proteins, enzymes, proteins involved in signal transduction, cytoskeletal proteins, and other proteins. Exemplary probes include, but are not limited to, polypeptides. Exemplary probes include, but are not limited to, proteins expressed from different alleles (similar polypeptides with slightly different amino acids) and different polypeptide conformations (similar polypeptides with different secondary and tertiary structures). In certain embodiments, a probe is chemically modified. Exemplary chemical modifications include, but are not limited to, modification by phosphorylation, methylation, and glycoslyation.

Exemplary probes include, but are not limited to, antibodies and receptor molecules. In certain embodiments, probes comprise antibodies directed to specific target polypeptide antigens.

Exemplary naturally occurring probes include, but are not limited to, signal molecules, such as hormones and other steroid-type molecules.

Exemplary probes include, but are not limited to, synthetic polypeptides, members of affinity sets, pharmaceuticals, and other organic small molecules.

In certain embodiments, a probe is a variant of a known transcriptional activator. Exemplary transcriptional activators include, but are not limited to, GAL4, VP16, Myc, Fos, Jun, and p53.

In certain embodiments, a probe is a variant of a known signal transduction or receptor molecule. Exemplary signal transduction molecules include, but are not limited to, Ras, Src, Bcr-Abl, FGF receptors, BMPs, and Rb.

In certain embodiments the probe is a variant of a conserved amino acid sequence. Exemplary conserved amino acid sequences include, but are not limited to, SH2 domains, SH3 domains, and PEST sequences.

In certain embodiments, probes are part of a probe set. The term "probe set" refers to a set of molecules comprising at least a probe. In certain embodiments, each molecule of a probe set may specifically interact with at least one other molecule of the probe set, which, under the proper conditions, may allow for the assembly of a complex of at least some molecules of the probe set. In certain embodiments, a probe set comprises (1) a polypeptide comprising a probe set nucleic acid binding domain and a probe, and (2) a probe set nucleic acid comprising a probe set polypeptide binding sequence, wherein the probe set polypeptide binding sequence is capable of binding to the probe set nucleic acid binding domain. In certain embodiments, a probe set may additionally comprise at least one additional molecule selected from at least one label, at least one separating moiety, and at least one addressable portion. In certain embodiments, a reaction composition comprises a plurality of different probe sets that comprise different probe set nucleic acid binding domains, different probe set nucleic acids, and different probes. In certain such embodiments, the reaction composition may also comprise some probe sets with the same probe set nucleic acid domain, the same probe set nucleic acid, and the same probe.

In certain embodiments, a probe set comprises (1) a polypeptide comprising a probe set nucleic acid binding domain and a probe, and (2) a probe set nucleic acid comprising a probe set polypeptide binding sequence, wherein the probe set polypeptide binding sequence is capable of binding to the probe set nucleic acid binding domain, and a complementary addressable portion, and (3) a separating moiety covalently linked to an addressable portion. Under the appropriate reaction conditions, the nucleic acid binding domain binds to the polypeptide binding sequence and the addressable portion hybridizes to the complementary addressable portion. Under certain such conditions, the probe is attached to the separating moiety through the probe set nucleic acid. In certain embodiments, a target specifically interacts with a probe such that the target is attached to the label through the probe and the probe nucleic acid. In certain such embodiments, the specific interaction between the target and the probe is detected by detecting the separating moiety attached to the target.

In certain embodiments, probes comprise aptamers, which are nucleic acids that specifically bind to certain polypeptide sequences.

In certain embodiments, a probe may comprise a member of a unique binding pair, such as streptavidin/biotin binding pairs, and affinity binding chemicals available from Prolinx™ (Bothell, WA) as exemplified, e.g., by U.S. Patent Nos. 5,831,046; 5,852,178; 5,859,210; 5,872,224; 5,877,297; 6,008,406; 6,013,783; 6,031,117; and 6,075,126.

### Certain Exemplary Labels

The term label" refers to any molecule that can be detected. In certain embodiments, a label can be a moiety that produces a signal or that interacts with another moiety to produce a signal. In certain embodiments, a label can interact with another moiety to modify a signal of the other moiety. In certain embodiments, a label can bind to another moiety or complex that produces a signal or that interacts with another moiety to produce a signal.

In various embodiments, a label is attached to a molecule and: (i) provides a detectable signal; (ii) interacts with a second label to modify the detectable signal provided by the second label, e.g., FRET (Fluorescent Resonance Energy Transfer); (iii) stabilizes hybridization, e.g., duplex formation; or (iv) provides a member of a binding complex or affinity set, e.g., affinity, antibody/antigen, ionic complexes, hapten/ligand, e.g., biotin/avidin.

In various embodiments, use of labels can be accomplished using any one of a large number of known techniques employing known labels, linkages, linking groups, reagents, reaction conditions, and analysis and purification methods. Labels include, but are not limited to, light-emitting or light-absorbing compounds which generate or quench a detectable fluorescent, chemiluminescent, or bioluminescent signal (see, e.g., Kricka, L. in Nonisotopic DNA Probe Techniques (1992), Academic Press, San Diego, pp. 3-28). Fluorescent reporter dyes useful as labels include, but are not limited to, fluoresceins (see, e.g., U.S. Patent Nos. 5,188,934; 6,008,379; and 6,020,481), rhodamines (see, e.g., U.S. Patent Nos. 5,366,860; 5,847,162; 5,936,087; 6,051,719; and 6,191,278), benzophenoxazines (see, e.g., U.S. Patent No. 6,140,500), energy-transfer fluorescent dyes, comprising pairs of donors and acceptors (see, e.g., U.S. Patent Nos. 5,863,727; 5,800,996; and 5,945,526), and cyanines (see, e.g., Kubista, WO 97/45539), as well as any other fluorescent moiety capable of generating a detectable signal. Examples of fluorescein dyes include, but are not limited to, 6-carboxyfluorescein; 2',4',1,4,-tetrachlorofluorescein; and 2',4',5',7',1,4-hexachlorofluorescein.

Other exemplary labels include, but are not limited to, luminescent molecules that emit light, and molecules that can be involved in luminescent reactions, such as luciferin-luciferase reactions, as a non-limiting example. Exemplary labels include, but are not limited to, chemiluminescent and electroluminescent molecules and reactions. As a non-limiting example, chemiluminescent labels may be exposed to film. Development of the film indicates whether or not targets are present in the sample or the quantity of the targets in the sample.

Exemplary labels include, but are not limited to, fluorescent proteins such as the Green Fluorescent Protein (GFP) and variants thereof.

Exemplary labels include, but are not limited to, donor-acceptor interactions, in which a donor molecule emits energy that is detected by an acceptor molecule. The acceptor molecule then emits a detectable signal.

Exemplary labels include, but are not limited to, molecules that are involved in infrared photon release.

Exemplary labels include, but are not limited to, quantum dots. "Quantum dots" refer to semiconductor nanocrystalline compounds capable of emitting a second energy in response to exposure to a first energy. Typically, the energy emitted by a single quantum dot always has the same predictable wavelength. Exemplary semiconductor nanocrystalline compounds include, but are not limited to, crystals of CdSe, CdS, and ZnS. Suitable quantum dots according to certain embodiments are described, e.g., in U.S. Pat. Nos. 5,990,479 and 6,207,392 B1, and in "Quantum-dot-tagged microbeads for multiplexed optical coding of biomolecules," Han et al., Nature Biotechnology, 19:631-635 (2001).

Exemplary labels include, but are not limited to, phosphors and radioisotopes. Radioisotopes may be directly detected, or may excite a fluorophore that emits a wavelength of light that is then detected. Phosphor particles may be excited by an infrared light (approximately around 980 nm) but emit signals within the visible spectrum, thus significantly reducing or eliminating background light.

Exemplary labels include, but are not limited to, particles with coded information, such as barcodes, and also include the microparticle tags described in U.S. Patent No. 4,053,433. Certain non-radioactive labeling methods, techniques, and reagents are reviewed in: Non-Radioactive Labeling, A Practical Introduction, Garman, A.J. (1997) Academic Press, San Diego.

Exemplary labels include, but are not limited to, a class of labels that effect the separation or immobilization of a molecule by specific or non-specific capture, for example biotin, digoxigenin, and other haptens (see, e.g., Andrus, A. "Chemical methods for 5' non-isotopic labeling of PCR probes and primers" (1995) in PCR 2: A Practical Approach, Oxford University Press, Oxford, pp. 39-54).

Labels may be "detectably different", which means that they are distinguishable from one another by at least one detection method. Detectably different labels include, but are not limited to, labels that emit light of different wavelengths, labels that absorb light of different wavelengths, labels that have different fluorescent decay lifetimes, labels that have different spectral signatures, labels that have different radioactive decay properties, labels of different charge, and labels of different size.

"Codeable label" refers to the one or more labels which are specific to a particular moiety. In certain embodiments, the moiety is a target and/or a probe. In embodiments in which a codeable label comprises more than one label, the labels may be the same or different. Detection of a given codeable label indicates the presence of the moiety to which the codeable label is specific. The absence of a given codeable label indicates the absence of the moiety to which the codeable label is specific.

In certain embodiments, the number of codeable labels is counted, which refers to the actual counting of individual codeable labels. Counting the number of codeable labels is distinguishable from analog signal detection, where an overall level of signal from multiple labels is detected. Analog signal detection typically uses integration of signals from multiple labels of the same type to determine the number of such labels present in a sample. For example, analog detection typically provides an estimate of the number of labels of a given type by comparing the brightness or level of intensity of the signal in the test sample to the brightness or level of intensity of the signal in controls with known quantities of the given labels.

Counting, by contrast, is a digital detection system in which the number of individual codeable labels is actually counted. Thus, in certain embodiments, if 200 of the same codeable labels are present in a sample, each of those labels is actually counted. In contrast, to determine the number of labels in a sample with analog detection, the overall signal from the 200 labels is measured and compared to the overall signal from known quantities of labels.

In certain embodiments, since it involves the actual counting of codeable labels, digital detection may be less influenced by background "noise," or incidental light that may be interpreted as part of the overall signal in analog detection.

In certain embodiments, one may determine fine distinctions between different numbers of codeable labels in different samples by counting the number of codeable labels. In contrast, the overall signal from multiple labels in analog detection, in certain instances, may be affected by the variable amount of background signal in different samples, which may obscure small differences in the number of labels in different samples.

In certain embodiments, where two or more detectably different codeable labels are being detected in a sample, possible inaccuracies due to overlapping signals from detectably different codeable labels may be minimized by counting each of the detectably different codeable labels. In certain analog detection methods, part of the signal from one label may be detected as signal from another different label, which may result in an inaccurate reading. This may be particularly the case if the signals from the different labels have overlapping emission ranges. By counting the individual codeable labels, in certain embodiments, one may minimize such inaccuracies that may sometimes result from analog detection where one measures the overall signal intensities from different labels.

In certain embodiments, the codeable labels are different sets of quantum dots that are specific for different probe sets, and the different sets of quantum dots are detectably different from one another.

In certain embodiments, the codeable labels are different sets of quantum dots that are specific for different target sets, and the different quantum dots are detectably different from one another. In certain embodiments, the codeable labels are different sets of quantum dots that are specific for different target groups, and the different quantum dots are detectably different from one another.

In certain embodiments, codeable labels may be attached directly to target sets. In certain embodiments, codeable labels may be attached directly to probe sets. In certain embodiments, labels may be attached to other molecules that are then attached to target sets. In certain embodiments, labels may be attached to other molecules that are then attached to probes sets. In certain embodiments, a codeable label may be attached to a molecule through a linking molecule, such as a chemical linkage group, or linking pair, e.g., a steptavidin-biotin linking pair. In certain embodiments, a codeable label may be attached to a target set prior to being added to a sample. In certain embodiments, a codeable label may be attached to a probe set prior to being added to a sample. In certain embodiments, a codeable label may become attached to a target set during the course of a reaction that forms a binding complex. In certain embodiments, a codeable label may become attached to a probe set during the course of a reaction that forms a binding complex.

In certain embodiments, labels are incorporated into beads, which may then be attached to target sets and/or probe sets. A "bead" refers to any material to which a target or probe can be attached. In various embodiments, beads may be of any shape, including, but not limited to, spheres, rods, cubes, and bars. In various embodiments, beads may be made of any substance, including, but not limited to, silica glass and polymers. In various embodiments, beads may be any size. Certain non-limiting examples of beads include those described, e.g., in U.S. Pat. Nos. 5,990,479 and 6,207,392 B1, and in "Quantum-dot-tagged microbeads for multiplexed optical coding of biomolecules," Han et al., Nature Biotechnology, 19:631-635 (2001).

In certain embodiments, beads comprise coated or uncoated particles comprising at least one material selected from magnetic material, paramagnetic material, silica glass, polyacrylamide, polysaccharide, plastic, latex, polystyrene, and other polymeric substances.

In certain embodiments, beads may comprise codeable labels, such as sets of quantum dots. Those skilled in the art are aware of certain suitable methods of obtaining beads with quantum dots. See, e.g., Han et al., Nature Biotechnology, 19:631-635 (2001), and U.S. Pat. No. 6,207,392. In certain embodiments, the quantum dots or other labels may be embedded in beads.

In certain embodiments, as a non-limiting example, quantum dots may be incorporated into cross-linked polymer beads. In certain embodiments, polystyrene beads may be synthesized using an emulsion of styrene (98% vol./vol.), divinylbenzene (1 % vol./vol.), and acrylic acid (1 % vol./vol.) at 70°C. In certain embodiments, the beads are then swelled in a solvent mixture containing 5% (vol./vol.) chloroform and 95 % (vol./vol.) propanol or butanol. In certain embodiments, a controlled amount of ZnS-capped CdSe quantum dots are added to the mixture. After incubation at room temperature, the embedding process is complete. In certain embodiments, the size of the beads may be controlled by the amount of a stabilizer (e.g., polyvinylpyrrolidone) used in the synthesis. In certain embodiments, a spherical bead 2 µm in diameter containing quantum dots that are 2-4 nm in diameter may contain tens of thousands of quantum dots.

In certain embodiments, the method of manufacturing beads discussed above may result in beads with varying numbers of quantum dots. Also, in certain embodiments, if one uses more than one color of quantum dot, one may obtain beads that have varying numbers of the different colors. In certain embodiments, after such bead preparation, the resulting beads are sorted by the relative number of quantum dots of each color in a given bead to obtain groups of identically labeled beads with distinct codeable labels. In certain embodiments, the sorting can be automated by machines, such as a Fluorescence Associated Cell Sorter (FACS) or other flow-cytometer type detection method that can distinguish between different codeable labels.

One of skill in the art will appreciate that there are many methods of obtaining beads comprising probes. Such methods include, but are not limited to, attaching the probes to the beads using covalent bonding, UV crosslinking, and linking through an affinity set. As a non-limiting example, streptavidin molecules may be covalently attached to the carboxylic acid groups on the bead surface. Oligonucleotide probes may be biotinylated, then linked to the beads via the streptavidin molecules.

### Certain Exemplary Binding Complexes

The term "binding complex" refers to a complex comprising a target set and a probe set.

In certain embodiments, the binding complex comprises additional molecules. For example without limitation, a binding complex may comprise a target set, a probe set, and one or more auxiliary polypeptides, which are additional polypeptides that are not members of the target set or probe set. In certain embodiments, the binding complex may comprise a target set, a probe set, one or more auxiliary polypeptides, and one or more additional molecules. Certain examples of additional molecules that may be part of the binding complex include, but are not limited to, nucleic acids, ligands, chemicals, and other molecules.

In certain embodiments, a binding complex may comprise one or more modifications to one or more components of the binding complex. In certain embodiments, a probe may be phosphorylated, pegylated, methylated, glycosylated, and/or otherwise modified. In certain embodiments, a target may be phosphorylated, pegylated, methylated, glycosylated, and/or otherwise modified. In certain embodiments, a modification may be utilized for formation of the binding complex. For example and not limitation, in certain embodiments, phosphorylation of a target polypeptide is utilized for binding complex formation.

In certain embodiments, binding complexes comprise one or more labels. In certain embodiments, binding complexes comprise one or more separation moieties.

In certain embodiments, the nucleic acids of the target set, the probe set, or both comprise multiple polypeptide binding sequences. In certain such embodiments, all of the polypeptide binding sequences on a nucleic acid are the same sequence. In certain embodiments, the nucleic acid comprises at least two polypeptide binding sequences that are different from one another.

Certain methods of forming binding complexes are known in the art. In certain embodiments, reaction compositions are designed using general biochemical techniques. Certain conditions under which polypeptide-polypeptide interactions occur are known in the art. Certain conditions under which polypeptides bind to DNA are also known in the art.

In certain embodiments, binding reactions are performed in conditions which mimic those inside a cell. In certain embodiments, a buffer used in a binding reaction contains at least one component selected from a salt (e.g., NaCl), a detergent (e.g., Nonidet P-40), and a buffering agent (e.g. 50 mM Tris-Cl (pH 8.0)). In certain embodiments, the concentration of salt and detergent is varied to increase or decrease the stringency of the binding conditions. In certain embodiments, a wash buffer is used after the binding reaction. In certain embodiments, the pH of a wash buffer is increased or decreased to vary the stringency of the wash. In certain embodiments, random nucleic acids (e.g., poly dl-dC or sheared Salmon Sperm DNA) are added to the reaction composition to soak up proteins that comprise non-specific nucleic acid binding domains.

In certain embodiments, the salt concentration may be increased or decreased to affect interactions between polypeptides. In certain embodiments, higher concentrations of salt are more disruptive of polypeptide-polypeptide interactions, leading to higher stringency. In certain embodiments, lower concentrations of salt are less disruptive of polypeptide-polypeptide interactions. In certain embodiments, the salt concentration in the buffer is titrated between a lower concentration of salt (e.g., 120 mM NaCl) and a high concentration of salt (e.g. 8 M NaCl), and an optimal salt concentration for the reaction conditions is determined.

According to certain embodiments, polypeptide-nucleic acid binding strength may be affected by varying MgCl₂ and/or cation concentrations. In certain embodiments, the concentration of MgCl₂ is titrated between 0.1 mM and 10 mM, and an optimum MgCl₂ concentration is determined. In certain embodiments, an optimum MgCl₂ concentration is between 0.1 mM and 10 mM MgCl₂. In certain embodiments, the cation concentration is less than 200 mM.

In certain embodiments, a binding complex is washed to test the stringency of the interaction between a probe and a target. In certain embodiments, a binding complex is washed under stringent conditions to test for the presence of strong interactions between a probe and a target. In certain embodiments, a binding complex is washed under non-stringent conditions to test for the presence of weak interactions.

### Certain Exemplary Detectable Complexes

The term "detectable complex" refers to a binding complex comprising at least one label.

In certain embodiments, a detectable complex is detected using a "detection set." The term "detection set" refers to a particular combination of labels and/or separating moieties that allows for detection of a particular detectable complex. For example and not limitation, a detection set may comprise two or more labels where the particular combination of labels is distinctive for a particular detection set. In certain embodiments, a single label may be part of multiple different detection sets.

According to certain embodiments, a detectable complex is produced when a target interacts with a probe and is not produced in the absence of interaction between a target and a probe. In certain embodiments, a detectable complex is formed if a target set and probe set specifically interact with one another. In certain embodiments, a detectable complex is formed when a label becomes attached to a separating moiety through the interaction of a target and a probe.

In certain embodiments, a detectable complex may be produced by antibody-antigen interactions. In certain embodiments, a detectable complex may be produced by aptamer-protein interactions. In certain embodiments, a detectable complex may be produced by interaction of specific binding pairs (e.g., a streptavidin-biotin interaction).

In certain embodiments, if a detectable complex is counted, then a target is present in the sample.

### Certain Exemplary Addressable Portions

The term "addressable portion" refers to an oligonucleotide sequence designed to hybridize to the complement of the addressable portion. For a pair of addressable portions that are complementary to one another, one member will be called an addressable portion and the other will be called a complementary addressable portion.

In certain embodiments, a binding complex comprises a first complementary addressable portion and a second complementary addressable portion. In certain such embodiments, the binding complex further comprises a first bead and a second bead. In certain embodiments, the first bead comprises a first addressable portion and the second bead comprises a second addressable portion. In certain embodiments, the first complementary addressable portion of the binding complex may hybridize to the first addressable portion of the first bead, and the second complementary addressable portion of the binding complex may hybridize to the second addressable portion of the second bead, to form a binding complex comprising the first bead and the second bead. In certain embodiments, the first bead, the second bead, or both beads comprise a separating moiety. In certain embodiments, the first bead, the second bead, or both beads comprise a codeable label.

In certain embodiments, restriction digest sites are incorporated into addressable portions. In certain embodiments, when an addressable portion hybridizes with a complementary addressable portion, a restriction digest site is reconstituted. In certain embodiments, when such a restriction digest site is reconstituted, a separating moiety or detectable bead may be released from a binding complex through restriction digest with an appropriate endonuclease. For example and not limitation, in certain embodiments, where the binding complex is attached to a magnet through a magnetic bead, the binding complex may be released from the magnetic bead by restriction digest. In certain embodiments, this type of release distinguishes binding complexes comprising labels from labels associated with the magnet through some other interaction. Thus, in certain embodiments, the restriction digest site increases the specificity of the detection assay.

### Certain Exemplary Separating Moieties

The term "separating" refers to any method used to separate a molecule from at least one other molecule. Exemplary methods of separation include, but are not limited to, separation based on density, size, electrical charge or ionic charge, diffusion, heat, flow cytometry, and directed light. In certain embodiments, separation may be achieved through the use of a separating moiety. A "separating moiety" refers to any moiety that, when attached to a second moiety, may be used to separate the second moiety from at least one other moiety in a reaction composition. In certain embodiments, when a separating moiety is included in a binding complex, it may be used to separate the binding complex from at least one other moiety in a reaction composition.

In certain embodiments, separation is achieved without any particular separating moiety incorporated in a detectable complex. In certain embodiments, methods that do not employ a specific separating moiety include, but are not limited to, separation based on density, size, electrical charge, or ionic charge, diffusion, heat, flow cytometry, and directed light. In certain embodiments, the detection of detectable complexes occurs without any separation of detectable complexes from other moieties.

In certain embodiments, a separating moiety is bound to a binding complex. Exemplary ways to bind a separating moiety to a second moiety include, but are not limited to, nucleotide hybridization, covalent attachment, affinity partner binding, and any other form of attachment sufficient to allow separation of the second moiety.

In certain embodiments, methods comprise separating a binding complex from separating moieties that are not in a binding complex, prior to the quantitating, or the detecting the presence or absence of, one or more targets. One of ordinary skill will appreciate that there are several methods that may be used according to certain embodiments for separating binding complexes from separating moieties not in a binding complex. As non-limiting examples in certain embodiments, differences in density or size of separating moieties may be used to separate binding complexes from separating moieties not in a binding complex. Exemplary methods of separation include, but are not limited to, use of sizing filters, sizing columns, density gradients, separation by gravity, and separation by centrifugation. Examples of such size-separating moieties include, but are not limited to, polymer beads.

For example, in certain embodiments, one may separate binding complexes comprising separating moieties from separating moieties not in a binding complex as follows. A composition may be formed comprising a separating bead and a detecting bead. The separating bead comprises a first codeable label, and the detecting bead comprises a second codeable label. The separating beads are smaller in size than the detecting beads. The separating bead is attached to a probe set. The detecting bead is attached to a target set. After binding complex formation, one can separate binding complexes from separating beads not in a binding complex based on the differences in size of the detecting beads in the binding complexes and the separating beads not in binding complexes. For example, one may pass the material through a sizing filter that allows separating beads to flow through and which retains binding complexes and detecting beads.

In certain embodiments, one may separate binding complexes from separating moieties not in binding complexes as follows. A composition may be formed comprising a separating bead and a detecting bead. The separating bead comprises a first codeable label, and the detecting bead comprises a second codeable label. The separating beads have a higher density than the detecting beads. The separating bead is attached to a probe set. The detecting bead is attached to a target set. After a binding complex is formed, one can separate binding complexes comprising a separating bead from separating beads not in a binding complex based on the differences in density of the detecting beads in the binding complexes and the separating beads not in a binding complex. For example, in certain embodiments, one may place the material in a density gradient, which will separate the binding complexes from the separating beads not in a binding complex. Also, in certain embodiments, gravity may be used to separate the binding complexes from the separating beads not in a binding complex. Thus, if the separating beads have a higher density than the detecting beads, the separating beads not in a binding complex will sink below the binding complexes that comprise both a separating bead and a detecting bead.

According to certain embodiments, other different properties of the separating moieties may be used. For example, in certain embodiments, one may use a separating moiety that has a particular property that attracts it to a particular position and other moieties in the reaction mixture that lack that property. For example, according to certain embodiments, the separating moiety may comprise a magnetic particle and the other moieties in the reaction mixture do not comprise a magnetic particle.

The term "magnetic particle" refers to material which can be moved using a magnetic force. This includes, but is not limited to, particles that are magnetized, particles that are not magnetized but are influenced by magnetic fields (e.g., colloidal iron, iron oxides (e.g., ferrite and magnetite), nickel, and nickel-iron alloys), and particles which can become magnetized (e.g., ferrite, magnetite, iron, nickel, and alloys thereof).

In certain embodiments, the magnetic particle comprises one or more materials selected from ferrite, magnetite, nickel, and iron, and the other moieties in the reaction mixture do not comprise such a material. In such embodiments, one can use such distinctive properties of the separating moiety to separate binding complexes that include a separating moiety from other moieties in the mixture that lack such distinctive properties.

Certain exemplary methods of separating binding complexes from other moieties include, but are not limited to, separation by density, separation by electrical charge, separation by drag coefficiencies (e.g., electrophoretic mobility), separation by diffusion or dialysis, and separation by heat or light (e.g., employing lasers to move labeled particles).

In certain embodiments, one may remove separating moieties, codeable labels, probe sets, or target sets not in binding complexes (free components) from a composition containing binding complexes prior to quantitating the target in the sample. In certain embodiments, one may remove binding complexes from a composition containing free components prior to the quantitating (or detecting the presence or absence of) the target in the sample.

In certain embodiments, separating the binding complex from free components comprises separating the binding complex from the sample.

As a nonlimiting illustration, in certain embodiments, one may detect the presence or absence of different binding complexes in a sample, such as a cell lysate, as follows. A sample is combined with a different detection set specific for each of the different binding complexes. Each detection set comprises a separating bead and a detecting bead. Each separating bead comprises a magnetic particle incorporated into the bead and a first addressable portion. Each separating bead also comprises a first codeable label that is specific for the first addressable portion. Each detecting bead comprises a bead and a second addressable portion. Each detecting bead also comprises a second codeable label that is specific for the second addressable portion. The first codeable label is detectably different from the second codeable label. The separating beads have a higher density than the detecting beads.

In this example, when the binding complex comprises both a complementary addressable portion to the first addressable portion of a given detection set and a complementary addressable portion to the second addressable portion of a given detection set, the addressable portions anneal to their corresponding complements to form a binding complex comprising the separating bead and the detecting bead.

In certain embodiments, the sample is then subjected to a density gradient such that binding complexes and detecting beads are situated above separating beads in the vessel. Binding complexes comprising both a separating bead and a detecting bead may then be separated from detecting beads not bound to binding complexes (free detecting beads) by a magnetic source. For example, one can remove the binding complexes from the sample containing the free detecting beads using the magnetic source, and can place the binding complexes in a separate vessel that does not contain any of the free detecting beads. One can then detect the presence or absence of binding complexes by counting the unique combinations of codeable labels.

In certain embodiments, binding complexes are formed comprising a magnetic bead comprising a first codeable label and a nonmagnetic bead comprising a second codeable label. In certain embodiments, an electromagnet is placed beneath a reaction vessel containing beads and binding complexes. When the electromagnet is turned on, binding complexes comprising at least one magnetic bead and free magnetic beads are attracted to the bottom of the vessel.

In certain embodiments, nonmagnetic beads that are not in a binding complex and other nonmagnetic moieties are removed by a continuous flow system comprising an input tube and an output tube. In certain embodiments, the electromagnet is then turned off, and the binding complexes and the magnetic beads that are not in a binding complex are then pulled out with a flow cytometer tube.

In certain embodiments, the binding complexes comprising at least one magnetic bead and the magnetic beads that are not in a binding complex are then sent through a flow cytometer and only combinations of the first and second codeable labels are counted. In such embodiments, the magnetic beads that are not in a binding complex will include only a first codeable label, which will not be counted.

In certain embodiments, one may carry out the method discussed above with a magnetic bead that does not include a codeable label. After separation of the nonmagnetic beads that are not in a binding complex, the binding complexes comprising at least one magnetic bead and the magnetic beads that are not in a binding complex are then sent through a flow cytometer. Since the magnetic beads do not have codeable label in such embodiments, only the codeable labels of the nonmagnetic beads in the binding complexes are counted.

In certain embodiments, binding complexes are formed comprising a magnetic bead, a nonmagnetic bead, and a codeable label. In certain embodiments, a first electromagnet is placed beneath a reaction vessel containing beads and binding complexes. When the first electromagnet is turned on, binding complexes and magnetic beads that are not in a binding complex are attracted to the bottom of the vessel.

In certain embodiments, nonmagnetic beads that are not in a binding complex and other nonmagnetic moieties are removed by a continuous flow system, comprising an input tube and an output tube. In certain embodiments, a vessel is used that may be inverted such that a substantial amount of liquid will not drain out when it is inverted. In certain embodiments, this may be accomplished using a small vessel in which surface tension inhibits drainage of liquid out of the vessel when the vessel is inverted. In certain embodiments that employ an inverted vessel, the vessel and the first electromagnet are then inverted and the first electromagnet is turned off. A second electromagnet is then turned on at the bottom of the inverted vessel to attract the binding complexes and the magnetic beads that are not in a binding complex. In certain embodiments, the binding complexes have more drag and less density than the magnetic beads that are not in a binding complex. Thus, in certain such embodiments, the magnetic beads that are not in a binding complex move faster than the binding complexes toward the second electomagnet. After the magnetic beads that are not in a binding complex are collected onto the second electromagnet, the vessel is inverted back before the binding complexes have reached the second electromagnet.

In certain embodiments, the binding complexes are then pulled out with a flow cytometer tube, and are sent through a flow cytometer. In certain such embodiments, the codeable labels of the binding complexes are counted.

In certain embodiments, one may carry out the method discussed above with a magnetic bead that does not include a codeable label.

In certain embodiments, binding complexes are formed comprising a magnetic bead, a nonmagnetic bead, and a codeable label. A filter is included in the vessel. In certain embodiments, the magnetic beads are designed such that they can pass through the filter and the nonmagnetic beads are designed such that they cannot pass through the filter. In certain embodiments, an electromagnet is placed beneath the reaction vessel containing beads and binding complexes. When the first electromagnet is turned on, binding complexes and magnetic beads that are not in a binding complex are attracted to the bottom of the vessel.

The magnetic beads that are not in a binding complex pass through the filter toward the magnet. The binding complexes are pulled toward the magnet, but cannot pass through the filter in view of the nonmagnetic bead of the complex. The binding complexes are held at the filter by the pull of the magnet. In certain embodiments, nonmagnetic beads that are not in a binding complex and other nonmagnetic moieties are then removed by a continuous flow system, comprising an input tube and an output tube. In certain embodiments, binding complexes can then be separated from magnetic beads that are not in a binding complex by moving the filter away from the electromagnet. Such movement of the filter pulls the binding complexes away from the electromagnet and away from the magnetic beads that are not in a binding complex.

In certain embodiments, binding complexes are then pulled out with a flow cytometer tube. In certain embodiments, the binding complexes are then sent through a flow cytometer and the codeable labels of the binding complexes are counted.

In certain embodiments, one may carry out the method discussed above with a magnetic bead that does not include a codeable label.

In certain embodiments, one may use grooves in a vessel that help to align binding complexes in a manner that facilitates the detection of the presence or absence of sets of labels. In certain embodiments, "aligned binding complexes" are complexes in which the separating beads of the complexes are closer to a given surface of a vessel than the detecting beads. For example, in certain embodiments, the separating beads may be smaller in size than the detecting beads. A groove is designed such that the separating beads fit into the groove, and the detecting beads are too large to fit into the groove. In certain embodiments, one may place a magnetic source near the groove in the vessel to attract and hold separating beads into the groove. One can then count the combinations of codeable labels to detect the presence or absence of binding complexes.

In certain embodiments, electrophoresis may also be used to separate separating moieties by charge or by a charge:mass ratio. In certain embodiments, a charged separating moiety may also be separated by ion exchange, e.g., by using an ion exchange column or a charge-based chromatography.

In certain embodiments, a separating moiety may also be a member of an affinity set.

In certain embodiments, separating moieties are separated in view of their mobility. In certain embodiments, separating in view of mobility is accomplished by the size of the separating moiety. In certain embodiments, mobility modifiers may be employed during electrophoresis. Exemplary mobility modifiers and methods of their use have been described, e.g., in U.S. Patent Nos. 5,470,705; 5,580,732; 5,624,800; and 5,989,871. In certain embodiments, by changing the mobility of a codeable label, one may distinguish signals associated with the presence of a binding complex from signals from labels not associated with the presence of a binding complex.

In certain embodiments, two or more different separating moieties or methods may be used. As a nonlimiting example, in certain embodiments, a binding complex may comprise a magnetic bead and a biotin-coated bead. A streptavidin-coated electromagnet is placed in the sample and turned on. The binding complexes and the magnetic beads that are not in a binding complex are attracted to the electromagnet. The biotin-coated beads in the binding complexes bind to the streptavidin on the electromagnet. The electromagnet is then turned off, and the magnetic beads that are not in a binding complex fall off the electromagnet, while the binding complexes remain bound to the electromagnet. In certain embodiments, the electromagnet is then removed from the sample with the binding complexes bound to the electromagnet, and the codeable labels in the binding complexes are detected by camera or scanner.

### Certain Exemplary Detection Methods

In certain embodiments, codeable labels are detected. In certain embodiments, codeable labels are counted.

As discussed above, counting of codeable labels refers to the actual counting of individual labels. In certain embodiments, detection and/or counting of labels includes identifying the code of a label if multiple detectably different labels are employed in the same procedure.

In various embodiments, codeable labels are detected with a type of flow cytometry, such as a Fluorescence Associated Cell Sorter (FACS), a Luminex™ detection device, or other technology developed for the detection of single codeable labels. In various embodiments, codeable labels are resolved by electrophoresis and detected during or after electrophoretic migration of the codeable labels. Electrophoresis includes, but is not limited to, capillary electrophoresis and field electrophoresis. In certain embodiments, such methods involve a device that excites the codeable labels (such as a laser, as a non-limiting example) and a scanning device that counts the codeable labels.

In certain embodiments, methods of detection involve static methods of detection. In certain embodiments, such methods involve placing binding complexes comprising codeable labels on a plate (as a non-limiting example), exciting the codeable labels with one or more excitation sources (such as lasers of different wavelengths, as a non-limiting example) and running a scanning device across the plate in order to count the codeable labels. In certain embodiments, the plate is moved back and forth across the field of detection of the scanning device. In certain embodiments, the codeable labels are attached to the plate or slide. In certain embodiments, a camera could image the entire field, and the image could be scanned in order to count the codeable labels.

According to certain embodiments, multiple targets may be detected in a sample, and distinguished by using different codeable labels. In certain embodiments, the codeable labels can be coded using two or more labels (e.g., in certain embodiments, quantum dots, fluorophores, or dyes are used). In certain embodiments, one may use multiple wavelengths or colors of labels, which multiplies the number of potential different codeable labels. For example, if a given codeable label is given a binary code, then one can detect the presence or absence of a specific color of label (either a "1" or "0" - hence a binary code). If only one binary color is used, then there are 2 codes, one with the label, and one without the label. If two binary colors are used (e.g., red and blue), then 4 codes are possible - (1) red, (2) blue, (3) red and blue, and (4) no color. Each additional color multiplies the number of possible codes by two. Thus, if 10 colors of labels are used, 1,024 binary codes are possible.

In certain embodiments, the codeable labels are incorporated or attached to beads.

Intensity may also be used as a factor in distinguishing codeable labels. In certain embodiments, intensity variations may be accomplished using codeable labels that include the same number of labels of a single wavelength, but different codeable labels have labels with different intensity levels. In certain embodiments, intensity variations may be accomplished by varying the number of labels of the same wavelength in different codeable labels attached to different beads. For example, in certain embodiments, one can use labels of the same wavelength in different codeable labels, and distinguish between the codeable labels using different numbers of labels in each different codeable label. For example, if a codeable label is given a ternary code (three levels of intensity for each color of label), then one color of label provides three possible codes - (1) no label, (2) one label, and (3) two labels. If two colors are used, then 9 ternary codes are possible. Six colors would allow 729 ternary codes.

Further, when codeable labels are attached to two different members of a detection set (for example, by incorporation into beads), the number of potential codes is further multiplied. For example, using two colors in a binary code, 16 different detection set codes are possible (4 X 4). Using two colors in a ternary code, 81 different detection set codes are possible (9 X 9). Using 10 colors in a binary code, over 1 million detection set codes are possible (1,024 X 1,024). Using 6 colors in a ternary code, over 500,000 detection set codes are possible (729 X 729).

In addition, in certain embodiments, the labels, such as quantum dots for example, are particularly efficient in transmitting a signal such that codeable label can be detected. In certain such embodiments, the codeable labels may be used to detect very few molecules within a sample without target amplification.

In certain embodiments, a detection set comprises a separating bead that comprises a separating moiety and a first codeable label; and comprises a detecting bead that comprises a second codeable label. In certain such embodiments, the first codeable label has a level of intensity that is specific for the separating bead. In certain such embodiments, the second codeable label has a level of intensity that is specific for the detecting bead. In certain embodiments, the beads of a detection set comprise labels of the same wavelength, but the first codeable label has a level of intensity that is specific for the separating bead, and the second codeable label has a level of intensity that is specific for the detecting bead.

In certain embodiments, the codeable label comprises at least 1,000 labels, wherein the labels have predetermined wavelengths that make one codeable label distinguishable from other codeable labels.

In certain embodiments, the codeable labels may comprise any number of labels from two to over 1,000. In certain embodiments, one uses codeable labels that allow one to detect the presence or absence of a particular target in a binding complex. In certain embodiments, one uses codeable labels such that the detection of the presence of a particular combination of labels confirms the presence of one specific binding complex. And, the detection of the absence of such a particular combination of labels confirms the absence of that one specific binding complex.

In certain embodiments, the labels are selected from quantum dots, phosphors, and fluorescent dyes.

In certain embodiments that employ a first bead and a second bead, both the separating bead and detecting bead of the detection set comprises a magnetic particle. In certain such embodiments, the beads are elongated and comprise a magnetic particle on one end and an addressable specific portion on the other end. In certain such embodiments, the beads further comprise labels placed in a particular order along the length of the bead. See, e.g., U.S. Patent No. 4,053,433, which describes elongated polymers with labels in particular orders.

In certain embodiments, the polarity or orientation of the magnetic particles in the beads is designed to facilitate alignment of the binding complexes. For example, in certain embodiments, the vessel containing the binding complexes will include a groove on a surface that is placed near a magnetic source. The beads are designed so that the polarity or orientation of the magnetic particles in the beads results in the binding complexes aligning in the groove with the first bead of each binding complex closer to one end of the groove than the second bead of that binding complex. One can then quantify binding complexes by quantitating the particular order of combinations of codeable labels.

In such embodiments, one can use a detection set that has a first bead and a second bead that comprise identical codeable labels, since the order of the identical codeable labels will be different on the first bead and on the second bead in the aligned binding complexes.

### Certain Exemplary Quantitation of Targets

In certain embodiments, one can quantitate targets. For example without limitation, one can quantitate binding complexes comprising targets. Quantitation can be applied to any of the methods discussed above with respect to detecting the presence or absence of targets. For example, and without limitation, one can quantitate the number of different binding complexes.

In certain embodiments, to quantitate the amount of a binding complex in a sample, one determines the amount of the particular combination of codeable labels for that binding complex.

Also, in certain embodiments that employ quantum dots as labels, the number of combinations of sets of quantum dots that are determined correlates directly to the actual number of binding complexes in a sample. Thus, in such embodiments, one need not compare the level of intensity of a fluorescent signal to a control signal to evaluate the number of binding complexes in the sample.

In certain embodiments, one detects the presence or absence of at least two different binding complexes in a sample. Each different binding complex differs from the other binding complexes by comprising a different probe set, a different target set, or both a different probe set and a different target set. In certain embodiments, one combines the sample with a different detection set specific for each of the different binding complexes, each detection set comprising (a) at least one separating bead, comprising a magnetic particle, a probe set-specific codeable label, and a probe set-specific addressable portion, wherein the probe set-specific codeable label and the probe-set specific addressable portion are specific for one of the different probe sets, and (b) at least one detecting bead, comprising a target set-specific codeable label, and a target set-specific addressable portion, wherein the target-set codeable label and the target-set specific addressable portion are specific for one of the different target sets. In certain embodiments, the method further comprises detecting the presence or absence of the at least two different binding complexes in the sample by counting the detectable complexes for each of the at least two target binding complexes. In certain embodiments, the method is identical to the method described above except that the detecting bead is associated with the probe set and the separating bead is associated with the target set.

In certain embodiments, by counting the unique combinations of codeable labels, one detects the presence or absence of particular binding complexes, which indicates the presence or absence of targets in the sample. Thus, in certain embodiments, one may determine the quantity of targets in a sample by determining the number of binding complexes.

### Certain Exemplary Two-Hybrid Systems

A two-hybrid system described by Fields et al. is a cell-based assay designed to detect polypeptide-polypeptide interactions. See e.g., Fields et al., Nature, 340, 245-246 (1989). That cell-based two-hybrid system uses two fusion polypeptides to activate a reporter gene. In that system, each fusion polypeptide comprises part of a transcription factor. If the two different fragments of the transcription factor are brought together by polypeptide-polypeptide interactions between the fusion polypeptides, the functional transcription factor is reconstituted, which activates transcription.

The version of the two-hybrid system described by Fields et al. typically is performed in a cell. Thus, it may be difficult to vary the cellular environment to evaluate the strength of the interactions between a target and a probe. Furthermore, that system relies on a reporter gene for detection. Typically, achieving expression of more than two distinguishable reporter genes in a cell is difficult. Furthermore, such cell-based two-hybrid systems are often designed to reduce the frequency of false positive results by using two separate reporter genes to test for a single polypeptide-polypeptide interaction. Consequently, in such instances, a cell-based two-hybrid system can only test for one or two sets of polypeptide-polypeptide interactions at a time. Additionally, in such assays, expression of the reporter gene is not measured digitally. Instead, typically the assay is a qualitative assay that measures only whether the reporter gene is expressed in a cell.

Certain cell-based two-hybrid systems are known in the art. Some examples of two-hybrid systems are described, e.g., in U.S. Patent Nos. 6,479,289; 5,667,973; 5,637,463; 5,283,173; and in Fields and Sternglanz, Trends Genet., 10, 286-292 (1994); Fields et al., Nature, 340, 245-246 (1989), Chien et al., Proc. Natl. Acad. Sci. USA 88, 9578-9582 (1991); Estojak et al., Mol. Cell. Biol. 15, 5820-5829 (1995); Allen et al., TIBS 20, 511-516 (1995); Bartel et al., Nature Genetics 12, 72-77 (1996); Bendixen et al., Nucleic Acids Research 22:9 1778-1779 (1994); Choi et al., Cell 78 499-512 (1994); Durfee et al. Genes & Devel. 7, 555-569 (1993); Finley and Brent, Proc. Natl. Acad. Sci. 91,12980-12984 (1994); Finley and Brent, Interaction trap cloning with yeast, in DNA Cloning 2: Expression Systems: A Practical Approach, Chapter 6, 169-203 Oxford University Press, (1995); Gyuris et al. Cell, 75, 791-803 (1993); Harper et al. Cell, 75, 805-816 (1993); Kranz et al. Genes & Devel. 8, 313-327 (1994). Certain two-hybrid systems used to identify small interacting peptides are described in, e.g., Yang et al., Nucleic Acids Res. 23, 1152-1156 (1995) and Colas et al., Nature 380, 548-550 (1996). Some of the effects of fusing exogenous polypeptides to nucleic acid binding domains are described , e.g., in Golemis and Brent, Mol. Cell. Biol. 12:7 3006-3014 (1992); Guarente and Ptashne, Proc. Natl. Acad. Sci. 78, 2199-2203 (1981); Lech et al. Cell 52, 179-184 (1988); Ma and Ptashne, Cell 51, 113-119 (1987). Certain transcriptional co-activators are discussed, e.g., in Guarente et al. TIBS 20, 517-521 (1995).

In certain embodiments, a two-hybrid system is performed *in vitro.* In certain embodiments, one fusion polypeptide comprises a probe, and another fusion polypeptide comprises a target. In certain embodiments, the probe is a variant of a known polypeptide. In certain embodiments, one then tests for targets that interact with the probe by testing for polypeptide-polypeptide interactions between targets and the probe. In certain embodiments, a plurality of targets are tested for polypeptide-polypeptide interactions with a single probe.

In certain embodiments, a probe fusion polypeptide comprises a nucleic acid binding domain and a probe. In certain embodiments, a target fusion polypeptide comprises a nucleic acid binding domain and a target. In certain such embodiments, the reaction composition comprises the probe fusion polypeptide and the target fusion polypeptide. In certain such embodiments, a binding complex may form between the probe fusion polypeptide and the target fusion polypeptide if there are polypeptide-polypeptide interactions between the probe and the target.

In certain embodiments, a reaction composition comprises a target set, a probe set, a codeable label attached to a first complementary addressable portion, and a separating moiety attached to a second complementary addressable portion. In certain such embodiments, the target set comprises: (a) a polypeptide comprising a target set nucleic acid binding domain and the target, and (b) a target set nucleic acid comprising a target set polypeptide binding sequence, wherein the target set polypeptide binding sequence is capable of binding to the target set nucleic acid binding domain. In certain embodiments, the target set nucleic acid also comprises a first addressable portion. In certain such embodiments, the probe set comprises: (a) a polypeptide comprising a probe set nucleic acid binding domain and the probe, and (b) a probe set nucleic acid comprising a probe set polypeptide binding sequence, wherein the probe set polypeptide binding sequence is capable of binding to the probe set nucleic acid binding domain. In certain embodiments, the probe set nucleic acid also comprises a second addressable portion. In certain embodiments, a binding complex may form between the probe set and the target set if there are polypeptide-polypeptide interactions between the probe and the target. In certain embodiments, the binding complex may comprise the label if the first addressable portion hybridizes to the first complementary addressable portion. In certain embodiments, the binding complex may comprise the separating moiety if the second addressable portion hybridizes to the second complementary addressable portion.

In certain embodiments, an *in vitro* two-hybrid system may be based on known polypeptides such that only target polypeptides suspected of interacting with a particular probe are tested. In certain embodiments, wherein the probe fusion polypeptide and the target fusion polypeptide are the only two fusion polypeptides in the reaction composition, a binding complex will only form if there are interactions between the probe and the target. In certain such embodiments, one may design both fusion polypeptides to test the interactions between the two polypeptides. In certain such embodiments, one may further test the interactions by creating variants of the probe fusion polypeptide, the target fusion polypeptide, or both fusion polypeptides. In various embodiments, the tests of the polypeptide-polypeptide interactions may test any aspect of the interaction. For example without limitation, in certain embodiments, one may test the interactions between a probe and a target by evaluating the binding affinity of various combinations of variants of the target, variants of the probe, or variants of both the target and the probe. Furthermore, in certain embodiments, one may evaluate the effect of a single amino acid change on the specificity of the interactions between a probe and a target by varying or deleting that single amino acid in that probe, that target, or both, and testing the specificity of those variants. Similarly, one may test other variants of polypeptides. In certain embodiments, the use of digital detection allows one to quantitate the number of targets that interact with probes, and thereby detect subtle differences in the specificity of different variants.

In certain embodiments, large screens may be conducted such that thousands, or even millions of targets may be tested for interactions with probes. In certain such embodiments, interactions between targets and probes may occur only between a small subset of probes and targets. Thus, in certain embodiments, no detectable interactions will occur between the majority of targets and any one probe. Furthermore, since multiple targets are tested at the same time, it is possible that multiple different binding complexes will form in the sample. Each different binding complex represents a different combination of a target and a probe. In certain embodiments, binding complexes may represent the detection of known polypeptides that were not previously known to interact with the probe. In certain embodiments, binding complexes may represent the detection of a previously unknown polypeptide that interacts with the probe.

In certain embodiments, no detectable binding complexes are formed. In certain embodiments, no detectable binding complexes are formed because no targets that interact with probes are present in the sample. This may occur, for example and not limitation, where the targets in a sample are randomly generated for use in a screen.

In certain embodiments where a large number of targets are screened, the targets may be partially or wholly generated from a cDNA library. Examples of cDNA libraries that may be used as sources for targets include, but are not limited to, cDNA libraries derived from particular organisms, cDNA libraries derived from particular tissues, cDNA libraries derived from particular developmental stages of an organism, cDNA libraries derived from particular developmental stages of particular tissues, cDNA libraries derived from particular cell lines, and any other library source or combination of library sources. In certain such embodiments, the targets may be generated from random DNA fragments encoding fragments of genes cloned in frame into a vector designed to express a target fusion polypeptide. In certain embodiments, targets are generated from genomic libraries or other libraries.

Certain methods of cloning and expressing cDNA libraries are known in the art. In certain embodiments, individual cDNAs are cloned into separate expression vectors immediately adjacent to a nucleic acid binding domain. In certain such embodiments, each individual cDNA is cloned in frame with the nucleic acid binding domain, such that a target fusion polypeptide results when the vector is expressed. In certain embodiments, each individual cDNA is cloned randomly next to the nucleic acid binding domain such that only some of the vectors result in the cDNA cloned in frame with the nucleic acid binding domain. Those cDNAs that are not cloned in frame may still be expressed and tested for interactions with a probe.

Certain other uses of a two-hybrid system are known in the art. In certain embodiments, a two-hybrid system may be used to identify inhibitors that reduce interactions between a target and a probe. Certain such molecules may include, but are not limited to, chemicals and polypeptide ligands. In certain embodiments, an inhibitor of interactions between a target and probe may be a polypeptide that interferes with the interactions by competing for binding of the target and/or probe. In certain embodiments, an inhibitor of interactions between a target and a probe may be a polypeptide that chemically modifies the target and/or probe thereby disrupting the interaction. For example without limitation, in certain embodiments, the inhibitor may be a kinase that phosphorylates an amino acid in a probe. When that particular amino acid is phosphorylated, binding of the probe to the target is reduced. Certain cell based two-hybrid systems designed to identify inhibitors of interactions between a target and probe are described, e.g., in U.S. Patent No. 5,525,490, and in Vidal et al., Proc. Natl. Acad. Sci. USA 93, 10315-10320 (1996); Vidal et al., Proc. Natl. Acad. Sci. USA 93, 10321-10326 (1996); Yasugi et al., J. Virol. 71, 5942-5951 (1997); Huang and Schreiber, Proc. Natl. Acad. Sci. USA 94, 13396-13401 (1997); and Shih et al., Proc. Natl. Acad. Sci. USA 93, 13896-13901 (1996).

In certain embodiments, a two hybrid system is used to detect inhibition of binding complex formation of a probe and a target. For example and not limitation, in certain embodiments, a reaction composition may comprise a probe set and a target set, wherein the probe set and the target set form a binding complex. In certain such embodiments, the reaction composition may further comprise one or more potential inhibitors of binding complex formation. In certain such embodiments, if an inhibitor reduces the interaction between a probe and a target, a reduced quantity of binding complexes may be detected. In certain embodiments, a reduced quantity of binding complexes may be detected as a threshold difference in signal values between a reaction composition comprising the inhibitor and a reaction composition without the inhibitor.

In certain embodiments, one may distinguish between specific inhibitors of binding complex formation and inhibitors which generally disrupt binding complex formation. The term "specific inhibitor of binding complex formation" refers to an inhibitor that reduces interactions between a target and a probe and does not reduce interactions between any other components of a binding complex. For example, and not limitation, some inhibitors may disrupt polypeptide-DNA interactions and/or may generally disrupt polypeptide-polypeptide interactions. One may wish to distinguish those inhibitors from specific inhibitors of binding complex formation. In certain such embodiments, one may distinguish between those two types of inhibitors by forming two or more different binding complexes in a sample. In the case of two different binding complexes, each different binding complex could comprise a different probe set, a different target set, or both a different probe set and a different target set. If the quantity of the first binding complex is reduced in the presence of an inhibitor, but the quantity of the second binding complex remains unchanged in the presence of the inhibitor, then one may conclude that the inhibitor specifically inhibits formation of the first binding complex by reducing probe-target interactions of the first binding complex. If the quantities of both the first and second binding complexes are reduced in the presence of the inhibitor, one may conclude that the inhibitor non-specifically reduces binding complex formation of those two different binding complexes.

In certain embodiments, a two hybrid system is used to detect one or more enhancers of binding complex formation of a probe and a target. For example and not limitation, in certain embodiments, a reaction composition may comprise a probe set and a target set, wherein the probe set and the target set form a binding complex. In certain such embodiments, the reaction composition may further comprise one or more potential enhancers of binding complex formation. In certain such embodiments, if one of the potential enhancers of binding complex formation increases the interaction between a probe and a target, an increased quantity of binding complexes may be detected. In certain embodiments, an increased quantity of binding complexes may be detected as a threshold difference in signal values between a reaction composition comprising the enhancer and a reaction composition without the enhancer.

In certain embodiments, a two-hybrid system may be used to test polypeptide-RNA interactions or polypeptide-chemical interactions. In certain such embodiments, a chemical or RNA molecule may be attached to a nucleic acid binding domain. In certain such embodiments, the chemical or RNA is used as a probe. Certain examples of two-hybrid systems using an RNA as a probe are described, e.g., in SenGupta et al., Proc. Natl. Acad. Sci. USA 93, 8496-8501(1996); Putz et al., Nucleic Acids Res. 24, 4838-4840 (1996); Wang et al., Nature 364, 121-126 (1996); Park et al., Proc. Natl. Acad. Sci. USA 96, 6694-6699 (1999); and SenGupta et al., RNA 5, 596-601 (1999). A non-limiting example of a two-hybrid system using a chemical ligand as a probe is described, e.g., in Licitra and Liu, Proc. Natl. Acad. Sci. USA 93, 12817-12821 (1996).

In certain embodiments of a two hybrid system, a nucleic acid may be covalently linked to a nucleic acid binding domain to test for polypeptide-nucleic acid interactions. In certain embodiments, the nucleic acid may be used as either a probe or a target. For example and not limitation, in certain embodiments, a nucleic acid molecule may be used as a probe to identify target polypeptides. In certain other embodiments, polypeptides may be used as probes to identify target nucleic acids. In certain embodiments, the interactions between a known nucleic acid and a known polypeptide may be tested by making variants of the nucleic acid, the polypeptide, or both and testing individual combinations of targets and probes. For example without limitation, one may evaluate the effect of a single nucleotide change in a nucleic acid sequence on the specificity of the interactions between a nucleic acid and a polypeptide by varying or deleting that single nucleotide in that probe and individually testing the specificity of those variants for the polypeptide. In certain embodiments, one may make more complex changes to the nucleic acid sequence. Exemplary changes include, but are not limited to, insertions, deletions, varying multiple nucleotides, and combinations of changes.

In certain embodiments, a chemical may be covalently attached to a nucleic acid binding domain to test for interactions between the chemical and one or more polypeptides. Exemplary chemicals include, but are not limited to, biological molecules (e.g., a steroid hormone), synthetic molecules (e.g., chemically synthesized drugs) and naturally occurring molecules. In certain embodiments, a polypeptide probe may be used to screen a library of chemical targets, wherein each chemical is individually attached to a nucleic acid binding domain. In certain embodiments, a chemical probe may be used to screen a library of polypeptide targets. In certain embodiments, the interactions between a known chemical and a known polypeptide may be tested by making variants of the chemical, the polypeptide, or both and testing different combinations of targets and probes. For example without limitation, in certain embodiments, one may evaluate the effect of a single amino acid change in a polypeptide on the specificity of the polypeptide for a chemical by altering or deleting the single amino acid and individually testing the specificity of those variants for the chemical.

In certain embodiments, a two-hybrid system may be used to identify interactions between polypeptides and aptamers. In certain embodiments, aptamers that interact with a probe may be selected from a random set of aptamers. In certain embodiments, aptamers may be designed to interact with a probe.

In certain embodiments, a two-hybrid system may comprise additional molecules that form a complex. In certain embodiments, a binding complex may comprise one or more auxiliary polypeptides. In certain embodiments, the auxiliary polypeptide may facilitate binding complex formation. In certain embodiments, the auxiliary polypeptide may not be necessary for probe-target interactions to occur, but may strengthen the interactions between the probe and target. Certain two-hybrid systems involving auxiliary polypeptides are described, e.g., in Tomashek et al., J. Biol. Chem. 271, 10397-10404 (1996); Zhang and Lautar, Anal. Biochem. 242, 68-72 (1996); Tirode et al., J. Biol. Chem. 272, 22995-22999 (1997); Kamada et al., Proc. Natl. Acad. Sci. USA 95, 8532-8537 (1998); Van Criekinge et al., Anal. Biochem. 263, 62-66 (1998) and Pause et al., Proc. Natl. Acad. Sci. USA 96, 9533-9538 (1999). In certain embodiments, a binding complex may contain an additional molecule, which may not be a polypeptide, but instead may be a chemical or other molecule. Certain two-hybrid systems using additional molecules are described in Chiu et al., Proc. Natl. Acad. Sci. USA 91, 12574-12578 (1994) and Lee et al., Mol. Endocrinol. 9, 243-254 (1995).

In certain embodiments, a two-hybrid system may be modified to use two different targets. In certain embodiments, the two different targets may be used in competitive assays to evaluate the interactions between the targets and a probe. Certain competitive two-hybrid assays using two targets are described, e.g., in Jiang and Carlson, Genes Dev. 10, 3105-3115 (1996); Inouye et al., Genetics 147, 479-492 (1997); Xu et al., Proc. Natl. Acad. Sci. USA 94, 12473-12478 (1997); Grossel et al., Nat. Biotechnol. 17, 1232-1233 (1999); and Serebriiskii et al., J. Biol. Chem. 274, 17080-17087 (1999).

In certain embodiments, the specificity of probe-target interactions may be evaluated using competitive two-hybrid assays. In certain embodiments, a reaction composition comprises one probe and two different targets. In certain embodiments, the quantity of probe is limited compared to the quantity of the two different targets. In certain embodiments, the two different targets compete to form binding complexes with the probe. In certain embodiments, one target is a variant of the other target. In certain embodiments, two distinguishable binding complexes may form in the reaction composition. In certain such embodiments, one binding complex corresponds to detection of interactions between the probe and one target. In certain such embodiments, the other binding complex corresponds to detection of interactions between the probe and the other target. In certain embodiments, the quantity of the two distinguishable binding complexes may be determined by digitally counting the individual binding complexes. In certain embodiments, the relative specificity of the two different targets for the probe may be determined by calculating the ratio of the two distinguishable binding complexes.

### Examples

The following prophetic examples are offered to illustrate certain embodiments.

### Example 1

This example describes a method for detecting one or more targets that interact with a probe from a pool of one hundred different targets. Each target is part of a target set that belongs to a single target group. Thus, there are one hundred different target sets in the target group. The target group is catalogued and subdivided into ten subgroups. These subgroups are designated subgroups one to ten. The method comprises forming ten reaction compositions, each reaction composition comprises a different subgroup. Thus, each reaction composition comprises ten different target sets of the target group. In addition to ten different target sets of the target group, each reaction composition comprises a probe set, a codeable label attached to a first complementary addressable portion, and a separating moiety (in the form of a magnetic bead) attached to a second complementary addressable portion.

The target group comprises a plurality of target sets, wherein each target set of the target group comprises: (a) a polypeptide comprising a target set nucleic acid binding domain and a target, and (b) a target set nucleic acid comprising a target set polypeptide binding sequence, wherein the target set polypeptide binding sequence is capable of binding to the target set nucleic acid binding domain. The target set nucleic acid also comprises a first addressable portion. The target group comprises a plurality of target sets that comprise the same target set nucleic acid binding domain and the same target set nucleic acid and comprise different targets.

The probe set comprises: (a) a polypeptide comprising a probe set nucleic acid binding domain and a probe, and (b) a probe set nucleic acid comprising a probe set polypeptide binding sequence, wherein the probe set polypeptide binding sequence is capable of binding to the probe set nucleic acid binding domain. The probe set nucleic acid also comprises a second addressable portion.

The reaction compositions are incubated under reaction conditions such that if a probe interacts with one of the one hundred different targets, a binding complex is formed. The binding complex comprises the target set comprising the target that interacts with the probe, the probe set, the codeable label, and the separating moiety.

A magnetic force is applied to the reaction composition to separate the binding complexes comprising a magnetic bead from those complexes and moieties that do not comprise a magnetic bead. This process separates out target sets that do not interact with a probe set since the magnetic bead is associated with the probe set. The remaining complexes include binding complexes comprising codeable labels and probe sets.

Following separation, the binding complexes are released from the magnet by digestion with a restriction endonuclease that cleaves between the magnetic bead and the polypeptide binding region of the second nucleic acid. Once the binding complexes are released, the remaining codeable labels are individually counted using a Fluorescence Associated Cell Sorter or similar device. Probe sets that do not interact with a target will not be counted since they will not comprise a codeable label. Thus, the number of codeable labels counted will accurately reflect the exact number of binding complexes formed in a reaction composition.

Those reaction compositions that produce binding complexes are subdivided to identify the target that interacts with the probe. Specifically, the ten target sets from the reaction composition that produced binding complexes are tested individually for interactions with the probe set in individual reaction compositions. Those reaction compositions that produce binding complexes contain an individual target that interacts with the probe. Because the targets are catalogued before distribution into reaction compositions, one can now go back to the catalogue of targets and identify the target that interacts with the probe.

It is possible that a screen of one hundred targets such as that described above will identify two or more targets that interact with a probe. By subdividing and testing reaction compositions that produce binding complexes, those different targets can be distinguished and identified. Furthermore, because the quantity of binding complexes is counted, one can compare the relative strengths of interaction between the probe and the identified targets.

Furthermore, the method used in this example can be scaled up or down depending on the number of targets to be tested.

### Example 2

This example describes a method for detecting at least one interaction between a probe and a target. The method comprises forming a reaction composition comprising a target set, a probe set, a codeable label attached to a first complementary addressable portion, and a separating moiety (in the form of a magnetic bead) attached to a second complementary addressable portion.

The target set comprises: (a) a polypeptide comprising a target set nucleic acid binding domain and the target, and (b) a target set nucleic acid comprising a target set polypeptide binding sequence, wherein the target set polypeptide binding sequence is capable of binding to the target set nucleic acid binding domain. The target set nucleic acid also comprises a first addressable portion.

The probe set comprises: (a) a polypeptide comprising a probe set nucleic acid binding domain and the probe, and (b) a probe set nucleic acid comprising a probe set polypeptide binding sequence, wherein the probe set polypeptide binding sequence is capable of binding to the probe set nucleic acid binding domain. The probe set nucleic acid also comprises a second addressable portion.

The reaction composition is incubated under reaction conditions such that the probe interacts with the target, forming a binding complex comprising the probe set, the target set, the codeable label, and the separating moiety.

A magnetic force is applied to the reaction composition to separate the binding complexes comprising a magnetic bead from those complexes and moieties that do not comprise a magnetic bead. This process separates out target sets that do not interact with a probe set since the magnetic bead is associated with the probe set. The remaining complexes include binding complexes comprising codeable labels and probe sets.

Following separation, the binding complexes are released from the magnet by digestion with a restriction endonuclease that cleaves between the magnetic bead and the polypeptide binding region of the second nucleic acid. Once the binding complexes are released, the remaining codeable labels are individually counted using a Fluorescence Associated Cell Sorter or similar device. Probe sets that do not interact with target will not be counted since they will not comprise a codeable label. Thus, the number of codeable labels counted will accurately reflect the exact number of binding complexes formed in the reaction composition.

### Example 3

This example describes a method for identifying one or more targets in a target group of one hundred targets, where the identified targets interact with a first probe, a second probe, or both the first and second probe. The method is similar to Example 1 except that two different probes are used. The two probes are tested for interactions with the target group in the same reaction composition. The method comprises subdividing a target group of 100 target sets into ten target subgroups as described in Example 1. Each subgroup is added to a different reaction composition. In addition to ten different target sets of the target group, each reaction composition comprises a first probe set, a second probe set, a codeable label attached to a first complementary addressable portion, a first separating moiety (in the form of a magnetic bead) attached to a first probe complementary addressable portion, and a second separating moiety (in the form of a magnetic bead) attached to a second probe complementary addressable portion. Both separating moieties comprise a codeable label. The codeable label attached to the first complementary portion is distinguishable from the codeable labels of the separating moieties. The codeable label of the first separating moiety is distinguishable from the codeable label of the second separating moiety.

The first probe set comprises: (a) a polypeptide comprising a first probe set nucleic acid binding domain and a first probe, and (b) a first probe set nucleic acid comprising a first probe set polypeptide binding sequence, wherein the first probe set polypeptide binding sequence is capable of binding to the first probe set nucleic acid binding domain. The first probe set nucleic acid also comprises a first probe set addressable portion.

The second probe set comprises: (a) a polypeptide comprising a second probe set nucleic acid binding domain and a first probe, and (b) a second probe set nucleic acid comprising a second probe set polypeptide binding sequence, wherein the second probe set polypeptide binding sequence is capable of binding to the second probe set nucleic acid binding domain. The second probe set nucleic acid also comprises a second probe set addressable portion.

Binding complexes are formed and separated as described in Example 1. Binding complexes comprising the first probe are distinguishable from binding complexes comprising the second probe due to the distinguishable codeable labels associated with the different separating moieties. Thus one can identify targets that interact with the first probe, the second probe, or both.

The complexity of this example can be increased by adding additional probes and/or target groups where each additional probe set and/or target group is associated with a distinguishable codeable label so that the different binding complexes are distinguishable.

### Example 4

This example describes a method for comparing the binding affinity of a first target for a probe with the binding affinity of a second target for the probe. The method comprises forming a reaction composition comprising two different target sets, a probe set, a first codeable label attached to a first complementary addressable portion, a second codeable label attached to a second complementary addressable portion, and a magnetic bead attached to a third complementary addressable portion. The first codeable label is distinguishable from the second codeable label.

The first target set comprises: (a) a polypeptide comprising a first target set nucleic acid binding domain and a first target, and (b) a first target set nucleic acid comprising a first target set polypeptide binding sequence, wherein the first target set polypeptide binding sequence is capable of binding to the first target set nucleic acid binding domain. The first target set nucleic acid also comprises a first addressable portion.

The second target set comprises: (a) a polypeptide comprising a second target set nucleic acid binding domain and a second target, wherein the first target and the second target are different, and (b) a second target set nucleic acid comprising a second target set polypeptide binding sequence, wherein the second target set polypeptide binding sequence is capable of binding to the second target set nucleic acid binding domain. The second target set nucleic acid also comprises a second addressable portion.

The probe set comprises: (a) a polypeptide comprising a probe set nucleic acid binding domain and a probe, and (b) a probe set nucleic acid comprising a probe set polypeptide binding sequence, wherein the probe set polypeptide binding sequence is capable of binding to the probe set nucleic acid binding domain. The probe set nucleic acid also comprises a third addressable portion.

The reaction composition is incubated under reaction conditions such that the first target set interacts with the probe set forming a first binding complex; the second target set interacts with the probe set forming a second binding complex; and the addressable portions hybridize to the corresponding complementary addressable portions. In certain embodiments, where the affinity of a first target for a probe is much greater than the affinity of a second target for a probe, and where the amount of first target is greater than the amount of probe, the reaction composition may comprise first binding complexes, but no second binding complexes.

A magnetic force is applied to the reaction composition to separate the binding complexes comprising a magnetic bead from those complexes and moieties that do not comprise a magnetic bead. This process separates out target sets that do not interact with a probe set since the magnetic bead is associated with the probe set. The remaining complexes include binding complexes comprising codeable labels and probe sets.

Following separation, the binding complexes are released from the magnet by digestion with a restriction endonuclease that cleaves between the magnetic bead and the polypeptide binding region of the second nucleic acid. Once the binding complexes are released, the remaining codeable labels are individually counted using a Fluorescence Associated Cell Sorter or similar device. Probe sets that do not interact with target will not be counted since they will not comprise a codeable label.

Since the first codeable label is distinguishable from the second codeable label, the quantity of first binding complexes and second binding complexes that form can be determined. By determining the number of binding complexes formed with the first target and comparing them to the number of binding complexes formed with the second target, one can compare the affinity of the two targets for the probe.

### Example 5

This example describes a method for detecting inhibition of binding complex formation by at least one potential inhibitor of binding complex formation. The method comprises forming two reaction compositions.

The first reaction composition comprises a target set, a probe set, a codeable label attached to a first complementary addressable portion, and a separating moiety (in the form of a magnetic bead) attached to a second complementary addressable portion.

The second reaction composition comprises the target set, the probe set, the codeable label attached to the first complementary addressable portion, the separating moiety attached to the second complementary addressable portion, and at least one potential inhibitor of binding complex formation.

The target set comprises: (a) a polypeptide comprising a target set nucleic acid binding domain and a target, and (b) a target set nucleic acid comprising a target set polypeptide binding sequence, wherein the target set polypeptide binding sequence is capable of binding to the target set nucleic acid binding domain;

The probe set comprises: (a) a polypeptide comprising a probe set nucleic acid binding domain and a probe, and (b) a probe set nucleic acid comprising a probe set polypeptide binding sequence, wherein the probe set polypeptide binding sequence is capable of binding to the probe set nucleic acid binding domain.

The first reaction composition is incubated under reaction conditions such that the first reaction composition forms a first test composition. If the probe interacts with the target in the first test composition, a binding complex comprising the probe set, the target set, the codeable label, and the separating bead is formed.

The second reaction composition is incubated under substantially the same reaction conditions as the first reaction composition, such that the second reaction composition forms a second test composition. If the probe interacts with the target in the second test composition, a binding complex comprising the probe set, the target set, the codeable label, and the separating bead is formed.

A magnetic force is applied to each test composition to separate the binding complexes comprising a magnetic bead from those complexes and moieties that do not comprise a magnetic bead. This process separates out target sets that do not interact with a probe set since the magnetic bead is associated with the probe set. The remaining complexes include binding complexes comprising codeable labels and probe sets.

Following separation, the binding complexes are released from the magnet by digestion with a restriction endonuclease that cleaves between the magnetic bead and the polypeptide binding region of the second nucleic acid. Once the binding Complexes are released, the remaining codeable labels are individually counted using a Fluorescence Associated Cell Sorter or similar device. Probe sets that do not interact with target will not be counted since they will not comprise a codeable label. The number of binding complexes that form in the first test composition is a first detectable signal value. The number of binding complexes that form in the second test composition is a second detectable signal value.

A threshold difference between the first detectable signal value and the second detectable signal value indicates inhibition of binding complex formation by the at least one potential inhibitor of binding complex formation. Furthermore, since the individual binding complexes that form are counted in determining the first detectable signal value and the second detectable signal value, one can judge the strength of inhibition by the number of binding complexes formed in the presence of the inhibitor compared to the number of binding complexes formed in the absence of the inhibitor.

## Claims

1. A method for detecting at least one target comprising:
forming a reaction composition comprising a target group and a probe set, wherein the target group comprises a plurality of target sets;
wherein each target set of the target group comprises: (a) a polypeptide comprising a target set nucleic acid binding domain and a target, and (b) a target set nucleic acid comprising a target set polypeptide binding sequence, wherein the target set polypeptide binding sequence is capable of binding to the target set nucleic acid binding domain;
wherein the target group comprises a plurality of target sets that comprise the same target set nucleic acid binding domain and the same target set nucleic acid and comprise different targets;
wherein the probe set comprises: (a) a polypeptide comprising a probe set nucleic acid binding domain and a probe, and (b) a probe set nucleic acid comprising a probe set polypeptide binding sequence, wherein the probe set polypeptide binding sequence is capable of binding to the probe set nucleic acid binding domain; and wherein the target set nucleic acid and the probe set nucleic acid comprise at least one addressable portion;
incubating the reaction composition under reaction conditions such that if the probe interacts with a target of a target set, a binding complex is produced, wherein the binding complex comprises the probe set and the target set;
detecting a label associated with the binding complex using one of the at least one addressable portions; and
detecting the target by detecting the label, wherein detecting the label comprises separating of the binding complex from the reaction composition,
wherein separating the binding complex comprises using the other one of the addressable portions.

2. A method detecting at least one interaction between a probe and a target comprising:
forming a reaction composition comprising a target set and a probe set;
wherein the target set comprises: (a) a polypeptide comprising a target set nucleic acid binding domain and the target, and (b) a target set nucleic acid comprising a target set polypeptide binding sequence, wherein the target set polypeptide binding sequence is capable of binding to the target set nucleic acid domain;
wherein the probe set comprises: (a) a polypeptide comprising a probe set nucleic acid binding domain and the probe, and (b) a probe set nucleic acid comprising a probe set polypeptide binding sequence, wherein the probe set polypeptide binding sequence is capable of binding to the probe set nucleic acid binding domain; and
wherein the target set nucleic acid and the probe set nucleic acid comprise at least one addressable portion;
incubating the reaction composition under reaction conditions such that if the probe interacts with the target, a binding complex is produced, wherein the binding complex comprises the probe set and the target set;
detecting a label associated with the binding complex using one of the at least one addressable portions; and
detecting the at least one interaction between the probe and the target by detecting the label,
wherein detecting the label comprises separating of the binding complex;
wherein separating the binding complex comprises using the other one of the addressable portions.

3. A method for detecting at least one target comprising:
forming a reaction composition comprising one or more target groups and one or more probe sets, wherein each target group comprises one or more target sets;
wherein each target set of each target group comprises: (a) a polypeptide comprising a target set nucleic acid binding domain and a target, and (b) a target set nucleic acid comprising a target set polypeptide binding sequence, wherein the target set polypeptide binding sequence is capable of binding to the target set nucleic acid binding domain;
wherein if a target group comprises multiple different target sets, the target group comprises a plurality of target sets that comprise the same target set nucleic acid binding domain and the same target set nucleic acid and comprise different targets;
wherein each probe set comprises: (a) a polypeptide comprising a probe set nucleic acid binding domain and a probe, and (b) a probe set nucleic acid comprising a probe set polypeptide binding sequence, wherein the probe set polypeptide binding sequence is capable of binding to the probe set nucleic acid binding domain; and
wherein the one or more target set nucleic acids and the one or more probe set nucleic acids comprise at least one addressable portion;
incubating the reaction composition under reaction conditions such that if a probe of a probe set interacts with a target of a target set, a binding complex is produced, wherein the binding complex produced comprises the probe set and the target set,
detecting a label associated with the binding complex using one of the at least one addressable portions; and
detecting the at least one target by detecting the label,
wherein detecting the label comprises separating of the binding complex from the reaction composition,
wherein separating the binding complex comprises using the other one of the addressable portions.

4. The method of claim 3, wherein the reaction composition comprises a plurality of different probe sets that comprise different probe set nucleic acid binding domains, different probe set nucleic acids, and different probes.

5. The method of claim 3, wherein two or more different binding complexes may be formed, wherein each different binding complex comprises a different combination of probe set and target set.

6. The method of claim 5, wherein at least one of the two or more different binding complexes may be distinguished from the other different binding complexes.

7. A method for comparing the binding affinities of two different targets with a probe comprising:
forming a reaction composition two different target sets and a probe set;
wherein the fist target set comprises: (a) a polypeptide comprising a first target set nucleic acid binding domain and a first target, and (b) a first target set nucleic acid comprising a first target set polypeptide binding sequence, wherein the first target set polypeptide binding sequence is capable of binding to the first target set nucleic acid binding domain;
wherein the second target set comprises: (a) a polypeptide comprising a second target set nucleic acid binding domain and a second target, wherein the first target and the second target are different, and (b) a second target set nucleic acid comprising a second target set polypeptide binding sequence, wherein the second target set polypeptide binding sequence is capable of binding to the second target set nucleic acid binding domain; and
wherein the probe set comprises: (a) a polypeptide comprising a probe set nucleic acid binding domain and a probe, and (b) a probe set nucleic acid comprising a probe set polypeptide binding sequence, wherein the probe set polypeptide binding sequence is capable of binding to the probe set nucleic acid binding domain;
wherein at least one of: a) both the first target set nucleic acid and the second target set nucleic acid comprise at least one addressable portion; and b) the probe set nucleic acid comprises at least one addressable portion;
incubating the reaction composition under reaction conditions such that a first binding complex may form; wherein the first binding complex comprises the first target set and the probe set; and wherein a second binding complex may form, wherein the second binding complex comprises the second target set and the probe set;
detecting a label associated with the first binding complexes that have been formed using at least one of the at least one addressable portions and detecting a label associated with the second binding complexes that have been formed using at least one of the at least one addressable portions; and
comparing the binding affinity of the first target to the probe with the binding affinity of the second target to the probe by determining a ratio of first binding complexes formed to second binding complexes formed,
wherein the detecting the label comprises separating at least one of the first binding complexes and the second binding complexes from the reaction composition;
wherein the separating at least one of the first binding complexes and the second binding complexes comprising using at least one of the at least one addressable portions.

8. The method of claim 7, wherein the first binding complexes are distinguishable from the second binding complexes.

9. A method for detecting inhibition of binding complex formation by at least one potential inhibitor of binding complex formation comprising:
forming a first reaction composition comprising a target set and a probe set, and forming a second reaction composition comprising the target set, the probe set, and at least one potential inhibitor or binding complex formation;
wherein the target set comprises: (a) a polypeptide comprising a target set nucleic acid binding domain and a target, and (b) a target set nucleic acid comprising a target set polypeptide binding sequence, wherein the target set polypeptide binding sequence is capable of binding to the target set nucleic acid domain;
wherein the probe set comprises: (a) a polypeptide comprising a probe set nucleic acid binding domain and a probe, and (b) a probe set nucleic acid comprising a probe set polypeptide binding sequence, wherein the probe set polypeptide binding sequence is capable of binding to the probe set nucleic acid binding domain; and
wherein the first nucleic acid and the second nucleic acid comprise at least one addressable portion;
incubating the first reaction composition under reaction conditions such that the first reaction composition forms a first test composition and incubating the second reaction composition under reaction conditions such that the second reaction composition forms a second test composition;
wherein if the probe interacts with the target in the first test composition, a binding complex is produced, wherein the binding complex comprises the probe set and the target set;
wherein if the probe interacts with the target in the second test composition, the binding complex is produced, wherein the binding complex comprises the probe set and the target set;
separating the binding complexes from the reaction composition using the other one of the addressable portions;
detecting a first detectable signal value from a label associated with the binding complexes that have been formed in the first test composition, and
detecting a second detectable signal value from a label associated with the binding complexes that have been formed in the second test composition;
wherein the detecting the first detectable signal value comprising using one of the at least one addressable portions and the detecting the second detectable signal value comprises using one of the at least one addressable portions; and
wherein a threshold difference between the first detectable signal value and the second detectable signal value indicates inhibition of binding complex formation by the at least one potential inhibitor of binding complex formation.

## Patentansprüche

1. Verfahren zur Detektion von zumindest eines Ziels umfassend:
Herstellen einer Reaktionszusammensetzung, die eine Zielgruppe und einen Sondensatz umfasst, wobei die Zielgruppe eine Vielzahl von Zielsätzen umfasst;
wobei jeder Zielsatz der Zielgruppe umfasst: (a) ein Polypeptid, das eine Zielsatz Nukleinsäure-Bindedomäne und ein Ziel, und (b) eine Zielsatznukleinsäure, die eine Zielsatz Polypeptidbindesequenz umfasst, wobei die Zielsatz Polypeptidbindesequenz in der Lage ist, an die Zielsatz Nukleinsäure-Bindedomäne zu binden;
wobei die Zielgruppe eine Vielzahl von Zielsätzen umfasst, die dieselbe Zielsatz Nukleinsäure-Bindedomäne und dieselbe Zielsatz Nukleinsäure umfassen und verschiedene Ziele umfassen;
wobei der Sondensatz umfasst: (a) ein Polypeptid, welches eine Sondensatz Nukleinsäure-Bindedomäne und eine Sonde umfasst, und (b) eine Sondensatznukleinsäure, die eine Sondensatz Polypeptidbindesequenz umfasst, wobei die Sondensatz Polypeptidbindesequenz in der Lage ist, an die Sondensatz Nukleinsäure-Bindedomäne zu binden; und wobei die Zielsatznukleinsäure und die Sondensatznukleinsäure zumindest einen adressierbaren Teil umfassen;
Inkubieren der Reaktionszusammensetzung unter solchen Reaktionsbedingungen, dass falls die Sonde mit einem Ziel eines Zielsatzes interagiert, ein Bindungskomplex produziert wird, wobei der Bindungskomplex den Sondensatz und den Zielsatz umfasst;
Detektieren einer Markierung, die mit dem Bindungskomplex assoziiert ist unter Verwendung des zumindest einen adressierbaren Teils; und
Detektieren des Ziels über das Detektieren der Markierung, wobei das Detektieren der Markierung die Trennung des Bindungskomplexes von der Reaktionszusammensetzung umfasst,
wobei die Trennung des Bindungskomplexes die Verwendung des anderen der adressierbaren Teile umfasst.

2. Verfahren zur Detektion von zumindest einer Wechselwirkung zwischen einer Sonde und einem Ziel umfassend:
Bildung einer Reaktionszusammensetzung, die einen Zielsatz und einen Sondensatz umfasst;
wobei der Zielsatz (a) ein Polypeptid umfassend eine Zielsatz Nukleinsäure-Bindedomäne und das Ziel, und (b) eine Zielsatznukleinsäure umfassend eine Zielsatz Polypeptidbindesequenz umfasst, wobei die Zielsatz Polypeptidbindesequenz in der Lage ist, an die Zielsatz Nukleinsäurebindedomäne zu binden;
wobei der Sondensatz (a) ein Polypeptid umfassend eine Sondensatz Nukleinsäurebindedomäne und die Sonde, und (b) eine Sondensatz Nukleinsäure umfassend eine Sondensatz Polypeptidbindesequenz umfasst, wobei die Sondensatz Polypeptidbindesequenz in der Lage ist, an die Sondensatz Nukleinsäurebindedomäne zu binden; und
wobei die Zielsatz Nukleinsäure und die Sondensatz Nukleinsäure zumindest einen adressierbaren Teil umfassen,
Inkubieren der Reaktionszusammensetzung unter solchen Reaktionsbedingungen, dass falls die Sonde mit einem Ziel eines Zielsatzes interagiert, ein Bindungskomplex produziert wird, wobei der Bindungskomplex den Sondensatz und den Zielsatz umfasst;
Detektieren einer Markierung, die mit dem Bindungskomplex assoziiert ist unter Verwendung des zumindest einen adressierbaren Teils; und
Detektieren des Ziels über das Detektieren der Markierung,
wobei das Detektieren der Markierung die Trennung des Bindungskomplexes von der Reaktionszusammensetzung umfasst;
wobei die Trennung des Bindungskomplexes die Verwendung des anderen der adressierbaren Teile umfasst.

3. Verfahren zur Detektion von zumindest einem Zeil umfassend:
Bildung einer Reaktionszusammensetzung, die einen oder mehrere Zielgruppen und einen oder mehrere Sondensätze umfasst, wobei jede Gruppe einen oder mehrere Zielsätze umfasst;
wobei jeder Zielsatz jeder Zielgruppe (a) ein Polypeptid umfassend eine Zielsatz Nukleinsäure-Bindedomäne und ein Ziel, und (b) eine Zielsatznukleinsäure umfassend eine Zielsatz Polypeptidbindesequenz umfasst, wobei die Zielsatz Polypeptidbindesequenz in der Lage ist, an die Zielsatz Nukleinsäurebindedomäne zu binden;
wobei falls eine Zielgruppe mehrere verschiedene Zielsätze umfasst, die Zielgruppe eine Vielzahl von Zielsätzen umfasst, die dieselbe Zielsatz Nukleinsäurebindedomäne und dieselbe Zielsatz Nukleinsäure umfassen, und die verschiedene Ziele umfassen;
wobei jeder Sondensatz (a) ein Polypeptid umfassend eine Sondensatz Nukleinsäurebindedomäne und eine Sonde, und (b) eine Sondensatz Nukleinsäure umfassend eine Sondensatz Polypeptidbindesequenz umfasst, wobei die Sondensatz Polypeptidbindesequenz in der Lage ist, an die Sondensatz Nukleinsäurebindedomäne zu binden; und
wobei die eine oder mehreren Zielsatz Nukleinsäuren und die eine oder mehreren Sondensatz Nukleinsäuren zumindest einen adressierbaren Teil umfassen;
Inkubieren der Reaktionszusammensetzung unter solchen Reaktionsbedingungen, dass falls die Sonde mit einem Ziel eines Zielsatzes interagiert, ein Bindungskomplex produziert wird, wobei der Bindungskomplex den Sondensatz und den Zielsatz umfasst;
Detektieren einer Markierung, die mit dem Bindungskomplex assoziiert ist unter Verwendung zumindest eines der adressierbaren Teile; und
Detektieren des zumindest einen Ziels über das Detektieren der Markierung,
wobei das Detektieren der Markierung die Trennung des Bindungskomplexes von der Reaktionszusammensetzung umfasst;
wobei die Trennung des Bindungskomplexes die Verwendung des anderen der adressierbaren Teile umfasst.

4. Das Verfahren nach Anspruch 3, wobei die Reaktionszusammensetzung eine Vielzahl von verschiedenen Sondensätzen umfasst, die unterschiedliche Sondensatz Nukleinsäurebindedomänen, unterschiedliche Sondensatz Nukleinsäuren und unterschiedliche Sonden umfasst.

5. Das Verfahren nach Anspruch 3, wobei zwei oder mehr unterschiedliche Bindungskomplexe von den anderen unterschiedlichen Bindungskomplexen unterschieden werden können.

6. Das Verfahren nach Anspruch 5, wobei zumindest einer der zwei oder mehr unterschiedlichen Bindungskomplexe von den anderen unterschiedlichen Bindungskomplexen unterschieden werden kann.

7. Verfahren zum Vergleich der Bindungsaffinität von zwei unterschiedlichen Zielen mittels einer Sonde, umfassend:
Bildung einer Reaktion, die zwei unterschiedliche Zielsätze und einen Sondensatz umfasst;
wobei der erste Zielsatz (a) ein Polypeptid umfassend eine erste Zielsatz Nukleinsäure-Bindedomäne und ein erstes Ziel, und (b) eine erste Zielsatznukleinsäure umfassend eine erste Zielsatz Polypeptidbindesequenz umfasst, wobei die erste Zielsatz Polypeptidbindesequenz in der Lage ist, an die erste Zielsatz Nukleinsäurebindedomäne zu binden;
wobei der zweite Zielsatz (a) ein Polypeptid umfassend eine zweite Zielsatz Nukleinsäure-Bindedomäne und ein zweites Ziel, und (b) eine zweite Zielsatznukleinsäure umfassend eine zweite Zielsatz Polypeptidbindesequenz umfasst, wobei die zweite Zielsatz Polypeptidbindesequenz in der Lage ist, an die zweite Zielsatz Nukleinsäurebindedomäne zu binden; und
wobei der Sondensatz (a) ein Polypeptid umfassend eine Sondensatz Nukleinsäurebindedomäne und eine Sonde, und (b) eine Sondensatz Nukleinsäure umfassend eine Sondensatz Polypeptidbindesequenz umfasst, wobei die Sondensatz Polypeptidbindesequenz in der Lage ist, an die Sondensatz Nukleinsäurebindedomäne zu binden;
wobei zumindest entweder a) sowohl die erste Zielsatz Nukleinsäure und die zweite Zielsatz Nukleinsäure zumindest einen adressierbaren Teil umfassen; und b) die Sondensatz Nukleinsäure zumindest einen adressierbaren Teil umfasst;
Inkubieren der Reaktionszusammensetzung unter solchen Reaktionsbedingungen, dass ein erster Bindungskomplex gebildet werden kann; wobei der erste Bindungskomplex den ersten Zielsatz und Sondensatz umfasst; und wobei ein zweiter Bindungskomplex gebildet werden kann, wobei der zweite Bindungskomplex den zweiten Zielsatz und Sondensatz umfasst,
Detektieren einer Markierung, die mit dem ersten Bindungskomplex assoziiert ist, der unter Verwendung von zumindest einem des zumindest einen adressierbaren Teils gebildet wurde und detektieren einer Markierung, die mit dem zweiten Bindungskomplex assoziiert ist, der unter Verwendung von zumindest einem des zumindest einen adressierbaren Teils gebildet wurde; und
Vergleichen der Bindungsaffinität des ersten Ziels zu der Sonde mit der Bindungsaffinität des zweiten Ziels zu der Sonde über die Bestimmung des Verhältnisses des ersten Bindungskomplexes der gebildet wurde zu dem zweiten Bindungskomplex der gebildet wurde,
wobei das Detektieren der Markierung die Trennung zumindest des ersten oder des zweiten Bindungskomplexes von der Reaktionszusammensetzung umfasst;
wobei die Trennung zumindest des ersten Bindungskomplexes oder des zweiten Bindungskomplexes die Verwendung zumindest eines des zumindest einen adressierbaren Teils umfasst.

8. Das Verfahren nach Anspruch 7, wobei die ersten Bindungskomplexe von den zweiten Bindungskomplexen unterscheidbar sind.

9. Verfahren zur Detektion der Inhibition der Bildung eines Bindungskomplexes durch zumindest einen potentiellen Inhibitor der Bindungskomplexbildung umfassend:
Bildung einer ersten Reaktionszusammensetzung umfassend einen Zielsatz und einen Sondensatz und Bildung einer zweiten Reaktionszusammensetzung umfassend den Zielsatz, den Sondensatz und zumindest einen potentiellen Inhibitor der Bindungskomplexbildung;
wobei der Zielsatz (a) ein Polypeptid umfassend eine Zielsatz Nukleinsäure-Bindedomäne und ein Ziel, und (b) eine Zielsatznukleinsäure umfassend eine Zielsatz Polypeptidbindesequenz umfasst, wobei die Zielsatz Polypeptidbindesequenz in der Lage ist, an die Zielsatz Nukleinsäurebindedomäne zu binden;
wobei der Sondensatz (a) ein Polypeptid umfassend eine Sondensatz Nukleinsäurebindedomäne und eine Sonde, und (b) eine Sondensatz Nukleinsäure umfassend eine Sondensatz Polypeptidbindesequenz umfasst, wobei die Sondensatz Polypeptidbindesequenz in der Lage ist, an die Sondensatz Nukleinsäurebindedomäne zu binden; und
wobei die erste Nukleinsäure und die zweite Nukleinsäure zumindest einen adressierbaren Teil umfassen;
Inkubieren der ersten Reaktionszusammensetzung unter solchen Reaktionsbedingungen, dass die erste Reaktionszusammensetzung eine erste Testzusammensetzung bildet und Inkubieren der zweiten Reaktionszusammensetzung unter solchen Reaktionsbedingungen, dass die zweite Reaktionszusammensetzung eine zweite Testzusammensetzung bildet;
wobei ein Bindungskomplex gebildet wird, falls die Sonde mit dem Ziel in der erten Testzusammensetzung interagiert, wobei der Bindungskomplex den Sondensatz und den Zielsatz umfasst;
wobei ein Bindungskomplex gebildet wird, falls die Sonde mit dem Ziel in der zweiten Testzusammensetzung interagiert, wobei der Bindungskomplex den Sondensatz und den Zielsatz umfasst;
Trennen der Bindungskomplexe von der Reaktionszusammensetzung unter Verwendung der anderen der adressierbaren Teile;
Detektieren eines ersten detektierbaren Signalswerts von einer Markierung, die mit den Bindungskomplexen assoziiert ist, die in der ersten Testzusammensetzung gebildet wurden, und
Detektieren eines zweiten detektierbaren Signalswerts von einer Markierung, die mit den Bindungskomplexen assoziiert ist, die in der zweiten Testzusammensetzung gebildet wurden;
wobei die Detektion des ersten detektierbaren Signalwerts die Verwendung von zumindest einem des zumindest einen adressierbaren Teils umfasst und die Detektion des zweiten detektierbaren Signalwerts die Verwendung von zumindest einem des zumindest einen adressierbaren Teils umfasst; und
wobei ein Schwellenwertunterschied zwischen dem ersten detektierbaren Signalwert und dem zweiten detektierbaren Signalwert die Inhibierung der Bindungskomplexbildung durch den zumindest einen potentiellen Inhibitor der Bindungskomplexbildung anzeigt.

## Revendications

1. Procédé de détection d'au moins une cible, comprenant :
la formation d'une composition réactionnelle comprenant un groupe de cibles et un jeu de sondes, le groupe de cibles comprenant une pluralité de jeux de cibles ;
dans lequel chaque jeu de cibles du groupe de cibles comprend : (a) un polypeptide comprenant un domaine de liaison d'acide nucléique de jeu de cibles et une cible, et (b) un acide nucléique de jeu de cibles comprenant une séquence de liaison polypeptidique de jeu de cibles, la séquence de liaison polypeptidique de jeu de cibles étant capable de se lier au domaine de liaison d'acide nucléique de jeu de cibles ;
dans lequel le groupe de cibles comprend une pluralité de jeux de cibles qui comprennent le même domaine de liaison d'acide nucléique de jeu de cibles et le même acide nucléique de jeu de cibles et comprennent des cibles différentes ;
dans lequel le jeu de sondes comprend : (a) un polypeptide comprenant un domaine de liaison d'acide nucléique de jeu de sondes et une sonde, et (b) un acide nucléique de jeu de sondes comprenant une séquence de liaison polypeptidique de jeu de sondes, la séquence de liaison polypeptidique de jeu de sondes étant capable de se lier au domaine de liaison d'acide nucléique de jeu de sondes ; et dans lequel l'acide nucléique de jeu de cibles et l'acide nucléique de jeu de sondes comprennent une ou plusieurs parties adressables ;
l'incubation de la composition réactionnelle dans des conditions réactionnelles telles que si la sonde interagit avec une cible d'un jeu de cibles, un complexe de liaison est produit, le complexe de liaison comprenant le jeu de sondes et le jeu de cibles ;
la détection d'un marqueur associé au complexe de liaison au moyen de la partie adressable ou d'une des parties adressables ; et
la détection de la cible en détectant le marqueur, la détection du marqueur comprenant la séparation du complexe de liaison de la composition réactionnelle,
dans lequel la séparation du complexe de liaison comprend l'utilisation d'une des autres parties adressables.

2. Procédé de détection d'au moins une interaction entre une sonde et une cible, comprenant :
la formation d'une composition réactionnelle comprenant un jeu de cibles et un jeu de sondes ;
dans lequel le jeu de cibles comprend : (a) un polypeptide comprenant un domaine de liaison d'acide nucléique de jeu de cibles et la cible, et (b) un acide nucléique de jeu de cibles comprenant une séquence de liaison polypeptidique de jeu de cibles, la séquence de liaison polypeptidique de jeu de cibles étant capable de se lier au domaine d'acide nucléique de jeu de cibles ;
dans lequel le jeu de sondes comprend : (a) un polypeptide comprenant un domaine de liaison d'acide nucléique de jeu de sondes et la sonde, et (b) un acide nucléique de jeu de sondes comprenant une séquence de liaison polypeptidique de jeu de sondes, la séquence de liaison polypeptidique de jeu de sondes étant capable de se lier au domaine de liaison d'acide nucléique de jeu de sondes ; et
dans lequel l'acide nucléique de jeu de cibles et l'acide nucléique de jeu de sondes comprennent une ou plusieurs parties adressables ;
l'incubation de la composition réactionnelle dans des conditions réactionnelles telles que si la sonde interagit avec la cible, un complexe de liaison est produit, le complexe de liaison comprenant le jeu de sondes et le jeu de cibles ;
la détection d'un marqueur associé au complexe de liaison au moyen de la partie adressable ou d'une des parties adressables ; et
la détection de la au moins une interaction entre la sonde et la cible en détectant le marqueur,
dans lequel la détection du marqueur comprend la séparation du complexe de liaison ;
dans lequel la séparation du complexe de liaison comprend l'utilisation d'une des autres parties adressables.

3. Procédé de détection d'au moins une cible, comprenant :
la formation d'une composition réactionnelle comprenant un ou plusieurs groupes de cibles et un ou plusieurs jeux de sondes, chaque groupe de cibles comprenant un ou plusieurs jeux de cibles ;
dans lequel chaque jeu de cibles de chaque groupe de cibles comprend : (a) un polypeptide comprenant un domaine de liaison d'acide nucléique de jeu de cibles et une cible, et (b) un acide nucléique de jeu de cibles comprenant une séquence de liaison polypeptidique de jeu de cibles, la séquence de liaison polypeptidique de jeu de cibles étant capable de se lier au domaine de liaison d'acide nucléique de jeu de cibles ;
dans lequel si un groupe de cibles comprend de multiples jeux de cibles différents, le groupe de cibles comprend une pluralité de jeux de cibles qui comprennent le même domaine de liaison d'acide nucléique de jeu de cibles et le même acide nucléique de jeu de cibles et comprennent des cibles différentes ;
dans lequel chaque jeu de sondes comprend : (a) un polypeptide comprenant un domaine de liaison d'acide nucléique de jeu de sondes et une sonde, et (b) un acide nucléique de jeu de sondes comprenant une séquence de liaison polypeptidique de jeu de sondes, la séquence de liaison polypeptidique de jeu de sondes étant capable de se lier au domaine de liaison d'acide nucléique de jeu de sondes ; et
dans lequel le ou les acides nucléiques de jeu de cibles et le ou les acides nucléiques de jeu de sondes comprennent une ou plusieurs parties adressables ;
l'incubation de la composition réactionnelle dans des conditions réactionnelles telles que si une sonde d'un jeu de sondes interagit avec une cible d'un jeu de cibles, un complexe de liaison est produit, le complexe de liaison produit comprenant le jeu de sondes et le jeu de cibles ;
la détection d'un marqueur associé au complexe de liaison au moyen de la partie adressable ou d'une des parties adressables ; et
la détection de la au moins une cible en détectant le marqueur,
dans lequel la détection du marqueur comprend la séparation du complexe de liaison de la composition réactionnelle ;
dans lequel la séparation du complexe de liaison comprend l'utilisation d'une des autres parties adressables.

4. Procédé selon la revendication 3, dans lequel la composition réactionnelle comprend une pluralité de différents jeux de sondes qui comprennent différents domaines de liaison d'acide nucléique de jeu de sondes, différents acides nucléiques de jeu de sondes et différentes sondes.

5. Procédé selon la revendication 3, dans lequel deux ou plus de deux complexes de liaison différents peuvent être formés, chaque complexe de liaison différent comprenant une combinaison différente de jeu de sondes et de jeu de cibles.

6. Procédé selon la revendication 5, dans lequel au moins un des deux ou plus de deux complexes de liaison différents peut être distingué des autres complexes de liaison différents.

7. Procédé de comparaison des affinités de liaison de deux cibles différentes avec une sonde, comprenant :
la formation d'une composition réactionnelle comprenant deux jeux de cibles différents et un jeu de sondes ;
dans lequel le premier jeu de cibles comprend : (a) un polypeptide comprenant un premier domaine de liaison d'acide nucléique de jeu de cibles et une première cible, et (b) un premier acide nucléique de jeu de cibles comprenant une première séquence de liaison polypeptidique de jeu de cibles, la première séquence de liaison polypeptidique de jeu de cibles étant capable de se lier au premier domaine de liaison d'acide nucléique de jeu de cibles ;
dans lequel le second jeu de cibles comprend : (a) un polypeptide comprenant un second domaine de liaison d'acide nucléique de jeu de cibles et une seconde cible, la première cible et la seconde cible étant différentes, et (b) un second acide nucléique de jeu de cibles comprenant une seconde séquence de liaison polypeptidique de jeu de cibles, la seconde séquence de liaison polypeptidique de jeu de cibles étant capable de se lier au second domaine de liaison d'acide nucléique de jeu de cibles ; et
dans lequel le jeu de sondes comprend : (a) un polypeptide comprenant un domaine de liaison d'acide nucléique de jeu de sondes et une sonde, et (b) un acide nucléique de jeu de sondes comprenant une séquence de liaison polypeptidique de jeu de sondes, la séquence de liaison polypeptidique de jeu de sondes étant capable de se lier au domaine de liaison d'acide nucléique de jeu de sondes ;
dans lequel au moins une des conditions suivantes est remplie : a) le premier acide nucléique de jeu de cibles et le second acide nucléique de jeu de cibles comprennent tous les deux au moins une partie adressable ; et b) l'acide nucléique de jeu de sondes comprend au moins une partie adressable ;
l'incubation de la composition réactionnelle dans des conditions réactionnelles telles qu'un premier complexe de liaison peut se former ; dans lequel le premier complexe de liaison comprend le premier jeu de cibles et le jeu de sondes ; et dans lequel un second complexe de liaison peut se former, le second complexe de liaison comprenant le second jeu de cibles et le jeu de sondes ;
la détection d'un marqueur associé aux premiers complexes de liaison qui ont été formés au moyen de la partie adressable ou d'une des parties adressables et la détection d'un marqueur associé aux seconds complexes de liaison qui ont été formés au moyen de la partie adressable ou d'une des parties adressables ; et
la comparaison de l'affinité de liaison de la première cible pour la sonde à l'affinité de liaison de la seconde cible pour la sonde en déterminant un rapport entre les premiers complexes de liaison formés et les seconds complexes de liaison formés,
dans lequel la détection du marqueur comprend une étape où au moins les premiers complexes de liaison et/ou au moins les seconds complexes de liaison sont séparés de la composition réactionnelle ;
l'étape où au moins les premiers complexes de liaison et/ou au moins les seconds complexes de liaison sont séparés utilisant au moins une des parties adressables.

8. Procédé selon la revendication 7, dans lequel les premiers complexes de liaison peuvent être distingués des seconds complexes de liaison.

9. Procédé de détection de l'inhibition de la formation d'un complexe de liaison par au moins un inhibiteur potentiel de formation de complexe de liaison, comprenant :
la formation d'une première composition réactionnelle comprenant un jeu de cibles et un jeu de sondes, et la formation d'une seconde composition réactionnelle comprenant le jeu de cibles, le jeu de sondes et au moins un inhibiteur potentiel de formation de complexe de liaison ;
dans lequel le jeu de cibles comprend : (a) un polypeptide comprenant un domaine de liaison d'acide nucléique de jeu de cibles et une cible, et (b) un acide nucléique de jeu de cibles comprenant une séquence de liaison polypeptidique de jeu de cibles, la séquence de liaison polypeptidique de jeu de cibles étant capable de se lier au domaine d'acide nucléique de jeu de cibles ;
dans lequel le jeu de sondes comprend : (a) un polypeptide comprenant un domaine de liaison d'acide nucléique de jeu de sondes et une sonde, et (b) un acide nucléique de jeu de sondes comprenant une séquence de liaison polypeptidique de jeu de sondes, la séquence de liaison polypeptidique de jeu de sondes étant capable de se lier au domaine de liaison d'acide nucléique de jeu de sondes ; et
dans lequel le premier acide nucléique et le second acide nucléique comprennent au moins une partie adressable ;
l'incubation de la première composition réactionnelle dans des conditions réactionnelles telles que la première composition réactionnelle forme une première composition de test et l'incubation de la seconde composition réactionnelle dans des conditions réactionnelles telles que la seconde composition réactionnelle forme une seconde composition de test ;
dans lequel si la sonde interagit avec la cible dans la première composition de test, un complexe de liaison est produit, le complexe de liaison comprenant le jeu de sondes et le jeu de cibles ;
dans lequel si la sonde interagit avec la cible dans la seconde composition de test, un complexe de liaison est produit, le complexe de liaison comprenant le jeu de sondes et le jeu de cibles ;
la séparation des complexes de liaison de la composition réactionnelle au moyen d'une des autres parties adressables ;
la détection d'une première valeur de signal détectable provenant d'un marqueur associé aux complexes de liaison qui ont été formés dans la première composition de test, et
la détection d'une seconde valeur de signal détectable provenant d'un marqueur associé aux complexes de liaison qui ont été formés dans la seconde composition de test ;
la détection de la première valeur de signal détectable comprenant l'utilisation d'une des parties adressables et la détection de la seconde valeur de signal détectable comprenant l'utilisation d'une des parties adressables ; et
une différence seuil entre la première valeur de signal détectable et la seconde valeur de signal détectable indiquant l'inhibition de la formation d'un complexe de liaison par le au moins un inhibiteur potentiel de formation de complexe de liaison.
